# EUROPEAN PATENT APPLICATION

(11) **EP 4 407 316 A1**
(43) Date of publication of application: **31.07.2024**
(21) Application number: 23153630.1
(22) Date of filing: 27.01.2023
(51) Int. Cl.: G01N 33/68

(54) **PROTEASOMAL DEGRADATION OF PROTEINS**

(71) Applicant: Katholieke Universiteit Leuven, KU Leuven R&D, 3000 Leuven (BE)
(72) Inventor: DAELEMANS, Dirk, 3400 Ezemaal (BE); KWANTEN, Bert, 3370 Boutersem (BE)

(57) **Abstract**

The invention relates to methods of identifying a compound that promotes ASB (Ankyrin repeat and SOCS box protein) mediated degradation of a target protein, comprising the step of:
- providing in vitro conditions allowing the binding of a compound and the protein target, and
- determining whether binding of the compound to the target protein results in the binding of an ASB protein to the target protein,
wherein a compound that results in the binding of an ASB protein to a target protein with the compound, is a candidate medicinal compound for conditions wherein a target protein is to be degraded.

## Description

### FIELD OF THE INVENTION

The invention relates to screening methods for compounds that promote proteasomal degradation of pathogenic protein.

The invention relates to ankyrin repeat and SOCS box (ASB) proteins and their use in these methods.

### BACKGROUND OF THE INVENTION

Despite the introduction of innovative mechanism-based treatment strategies such as targeted therapy and immuno-oncology, inherent and acquired drug resistance remains a major limitation for long-term cancer control. To address these issues, the current paradigm states that combination therapy should be the best treatment option, as targeting more than one cancer hallmark hinders development of resistance¹. Another element to avoid poor clinical response, is the development of predictive biomarkers which allow devising a treatment regimen tailored to the individual patient². Both strategies rely on a profound insight into the tumor's genetics and signaling pathways as well as into the pharmacogenetic properties of the treatment.

The advent of CRISPR-Cas genome editing and its adaptation into pooled libraries, offers a powerful tool to systematically identify genetic dependencies in human cells that modulate activity of a chemical compound³⁻⁷. To date, the majority of screens that combine drug treatment with CRISPR-Cas knockout have been positive screens i.e., screens that aim at enriching resistant cells and uncovering resistance mechanisms. Fewer studies have reported negative screening, which identifies genes that enhance cell killing⁸⁻¹⁵. As these screens uncover synthetic lethal drug-gene interactions, they have the potential to expose new drug target combinations. The execution of such negative screens can be more complicated as these require prolonged selection with an adequate sub-lethal dose of the drug while maintaining a parallel, non-selected control. On a more fundamental level, CRISPR screening may also provide new insights into the basic mechanism of action of a drug, previously unknown gene functions and functional relationships between genes.

In recent years, pharmacologic inhibition of the XPO1-mediated nuclear export pathway has been validated as a potent, targeted anti-cancer therapy. XPO1, the main effector of nuclear export, mediates transport of cargo proteins bearing a nuclear export signal (NES)¹⁶. These include a large number of tumor suppressor and oncogenic proteins; therefore, nuclear export plays an essential part in balancing cell cycle progression and cellular death mechanisms. Overexpression of XPO1 and aberrant subcellular cargo localization have been observed in different cancer types and correlate with poor prognosis and drug resistance, thus establishing XPO1 as a cancer drug target¹⁷. The discovery of drug-like N-azolyacrylate derived small-molecule inhibitors of XPO1 and their subsequent optimisation through molecular modelling, led to the development of a new class of drugs named Selective Inhibitors of Nuclear Export (SINE)¹⁸⁻²⁰. SINE bind covalently into the cargo-binding cleft of XPO1, which interferes with cargo docking and blocks nuclear export^{21,22}. This results in accumulation of tumor suppressors in the nucleus of cancer cells and restores their proper function, resulting in cell cycle arrest and apoptosis. SINE have demonstrated potent anticancer activity in several pre-clinical cancer models²³⁻²⁵. Furthermore, the lead SINE clinical candidate, selinexor, has recently obtained FDA, EMA and China's NMPA approval for treatment of relapsed or refractory multiple myeloma and diffuse large B-cell lymphoma (DLBCL)²⁶⁻²⁹ and is subject to ongoing clinical trials in solid tumor malignancies.

In the clinic, selinexor performs best when used in combination with other established drugs such as dexamethasone, proteasome inhibitors (bortezomib, carfilzomib), and immunomodulators (lenalidomide, pomalidomide). Moreover, a number of preclinical studies have reported synergistic drug interactions with SINE and their capacity to overcome several forms of acquired resistance³⁰⁻⁴⁴, providing further evidence that their unique mechanism of action renders this class of drugs exceptionally amenable to combination therapy. Since direct screening of all possible drug combinations is unfeasible, the use of CRISPR-Cas screening provides a general, non-biased strategy to uncover new synthetic lethal drug-gene interactions.

Predicting which patients will respond to treatment with selinexor is an essential part of developing a successful and effective therapy. Until now, only a handful biomarkers of response to selinexor have been suggested⁴⁵⁻⁴⁹ and these require further validation based on newly emerging patient data from clinical studies. While biomarkers have been demonstrated to improve clinical outcome for targeted therapies², their development requires a thorough understanding of the drug's mechanism of action. In this respect, high-throughput CRISPR-Cas screening for genes that modulate sensitivity to selinexor could add mechanistic insight and aid with the interpretation of patient data.

### SUMMARY OF THE INVENTION

In this study, we performed genome-wide CRISPR-SpCas9 knockout screening with selinexor in chronic myelogenous lymphoma, multiple myeloma and DLBCL cell lines. By prolonged screening at a moderate concentration (IC₅₀), we were able to identify genes that upon disruption either confer increased resistance or susceptibility to selinexor. We observe substantial heterogeneity between cell lines, reflecting the complex interaction network of XPO1 and the diverse, context-specific effects of XPOL inhibition. In multiple myeloma, the TGFβ-SMAD4 pathway emerged as an important mediator of resistance which was also predictive of progression-free survival in patients. We focused our attention on ASB8 (ankyrin and SOCS box protein 8), the strongest common sensitizer in all three cancer types. ASB8 is a poorly characterized substrate recognition unit in a subset of E3 ubiquitin ligase complexes for which the physiological targets remain largely unknown⁵⁰. We demonstrate that ASB8 triggers proteasomal degradation of selinexor-bound XPO1 and propose that XPO1 is a substrate of ASB8.

The invention is further summarized in the following statements:
1. A method of identifying a compound that promotes ASB (Ankyrin repeat and SOCS box protein) mediated degradation of a target protein, comprising the step of:
   - providing in vitro conditions allowing the binding of a compound and the protein target, and
   - determining whether binding of the compound to the target protein results in the binding of an ASB protein to the target protein,
   wherein a compound that results in the binding of an ASB protein to a target protein with the compound, is a candidate medicinal compound for conditions wherein a target protein is to be degraded.
2. The method according to statement 1, wherein the ASB is ASB8.
3. The method according to statement 1 or 2, further comprising the step of testing whether the binding of the compound to the target protein inhibits the function of the target protein.
4. The method according to any one of statements 1 to 3, which determining the binding of an ASB protein to a target protein is performed with a co-immune precipitation assay with an antibody against the target protein, and wherein is determined whether the ASB protein is co-precipitated.
5. The method according to any one of statements 1 to 3, wherein binding between an ASB protein and a target protein are determined by a method selected from the group consisting of native mass spectrometry, FRET, HTFR and a thermal shift assay.
6. The method according to any one of statements 1 to 3, wherein a target protein is selected by a method comprising the steps of:
   - providing a cell line wherein the ASB gene is inactivated and a cell line wherein the ASB protein is overexpressed,
   - treating the cell lines with a promiscuous metabolite,
   - determining the identity and concentration of proteins in the treated cell lines, wherein a protein, which is absent in the ABS overexpressing cells or has a lower concentration in the ABS overexpressing cells, is candidate target protein.
7. The method according to statement 6, wherein the promiscuous metabolite is 4-octyl-itacanoate.
8. The method according to any one of statements 1 to 3, comprising the step of
   - providing a cell line wherein ASB gene is inactivated and a cell line wherein ASB protein is overexpressed,
   - adding a compound to the cell lines, and
   - determining a change in the phenotype of one of the cell lines, wherein a change of phenotype is indicative of the compound being a compound promoting ASB mediation degradation.
9. The method according to statement 8, further determining the target protein that binds the compound added to the cell lines.
10. The method according to statement 9, further determining whether the compound modified or inhibits the function of the target protein.

### DETAILED DESCRIPTION

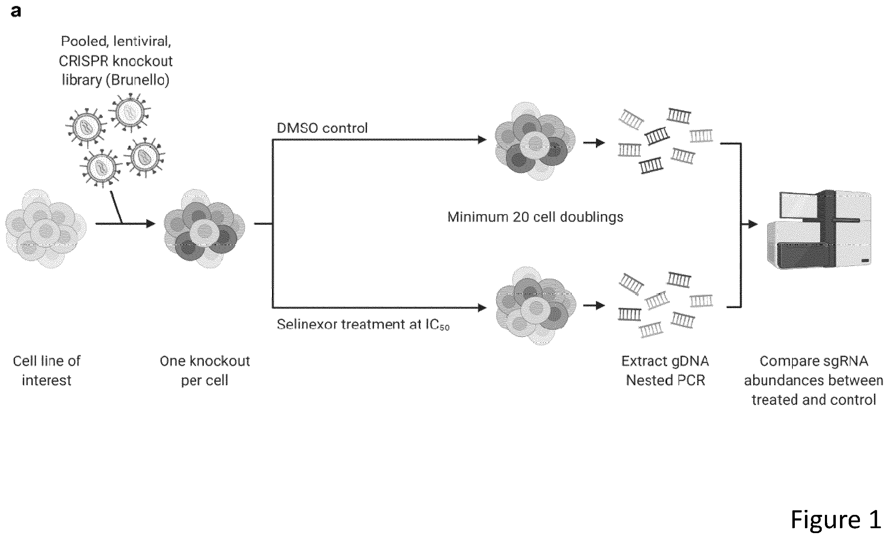

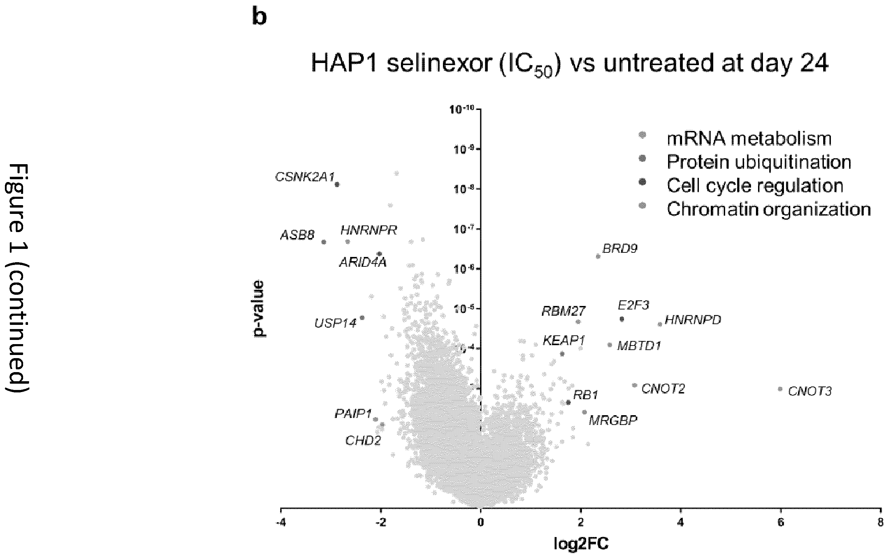

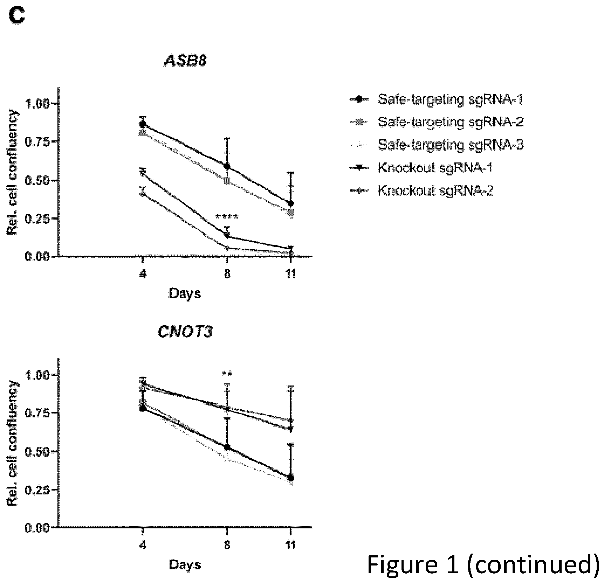

***Figure 1*****.** Genome-wide knockout screen in HAP1 (chronic myeloid leukemia) cells with selinexor. **a** Experimental workflow for CRISPR-Cas9 knockout screening, **b** Gene-level comparison of drug-treated group versus control group after 24 days of selection with 100 nM selinexor (IC₅₀). Log₂(fold change) (log2FC) on the x axis represents the averaged difference of the four sgRNAs for each gene between the two groups. One-sided p-values on the y axis were calculated with CRISPRBetaBinomial⁵² (*See* **Methods**)**. c** Individual validation of hits with a cell confluency assay. Cells were transduced with a vector encoding either a single-knockout or safe-targeting sgRNA and selected with puromycin. Next, knockout and control cells were treated with 100 nM selinexor or DMSO over a period of eleven days. Cell confluency was measured three times and normalized to the untreated DMSO condition. Data were collected from three repeated experiments. Error bars denote mean+SD. p-values were calculated with a two-tailed t-test by aggregating data points from safe-targeting sgRNAs and those from gene-targeting sgRNAs at day eight (*-<0.05; **-<0.01, ***-<0.001; ^{∗∗∗∗}- <0.0001).

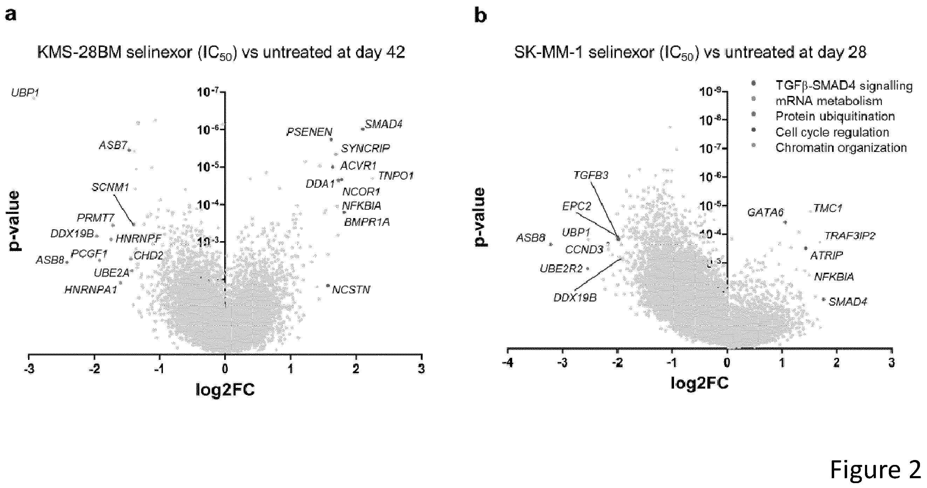

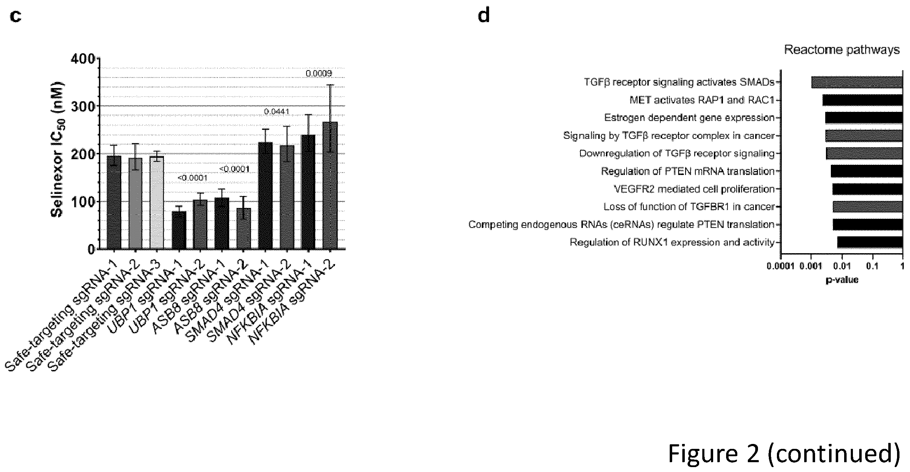

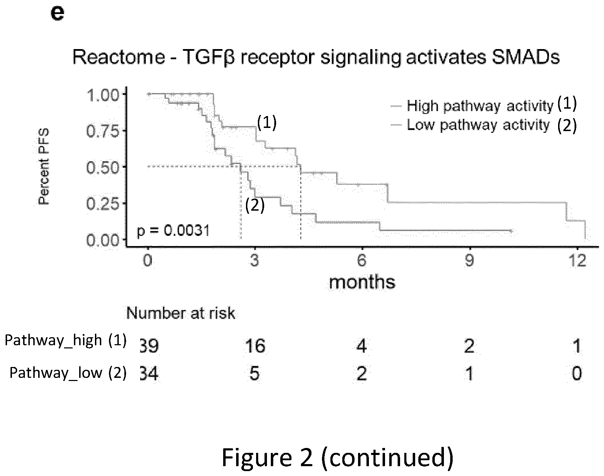

***Figure 2******.*** Genome-wide knockout screen with selinexor in multiple myeloma cell lines. **a-b** Gene-level comparison of drug-treated group versus control group after selection with selinexor (IC₅₀) for 42 days in KMS-28BM (**a**) and 28 days in SK-MM-1 cells (**b**). Log₂(fold change) (log2FC) on the x axis represents the averaged difference of the four sgRNAs for each gene between the two groups. One-sided p-values on the y axis were calculated with CRISPRBetaBinomial⁵² (*See* **Methods**). **c** Selinexor IC₅₀ values from KMS-28BM cells transduced with a safe-targeting or gene-targeting sgRNA. After six days of treatment with a dilution series of selinexor, cell viability was measured by MTS assay and data were normalized to untreated cells. IC₅₀ values were determined by fitting a non-linear, four-parameter regression curve with Graphpad Prism 9. Combined data from safe-targeting sgRNAs were compared with those from gene-targeting sgRNAs using an extra sum-of-squares F-test. p-values for difference in IC₅₀ are given on bar chart. Data were obtained from two separate experiments performed in triplicate. **d** Top Reactome pathways correlating gene expression data from patients enrolled in the STORM study with progression-free survival (PFS) upon treatment with selinexor and dexamethasone. Gene sets within the "TGFβ signaling pathway" group are shown in red. **e** Comparison of PFS between patients with high or low activity levels of the Reactome "TGFβ receptor signaling activates SMADs" pathway . *See* **Methods** for a detailed description.

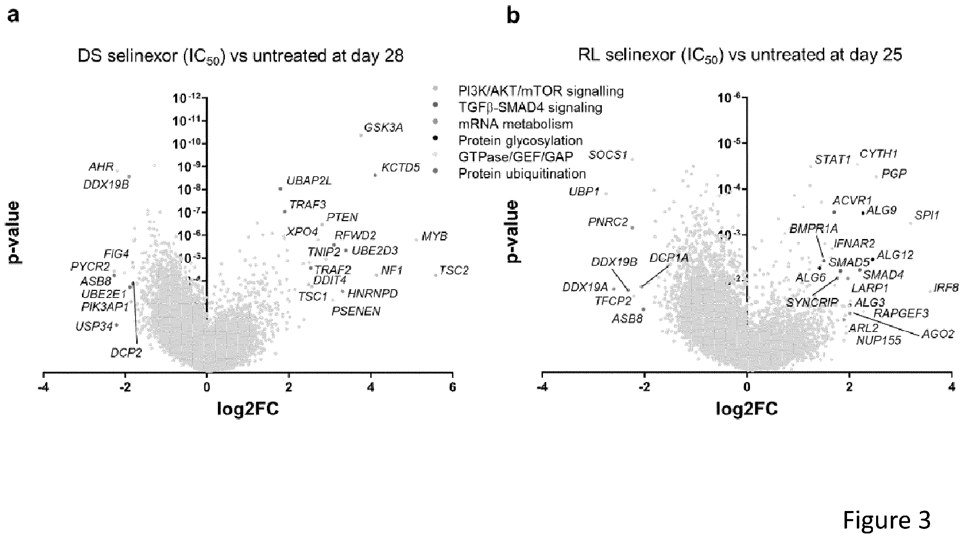

***Figure 3******.*** Genome-wide knockout screen with selinexor in DLBCL cell lines. **a-b** Gene-level comparison of drug-treated group versus control group after selection with selinexor (IC₅₀) for 28 days in DS (**a**) and 25 days in RL cells (**b**). Log₂(fold change) (log2FC) on the x axis represents the averaged difference of the four sgRNAs for each gene between the two groups. One-sided p-values on the y axis were calculated with CRISPRBetaBinomial⁵² (*See* **Methods**).

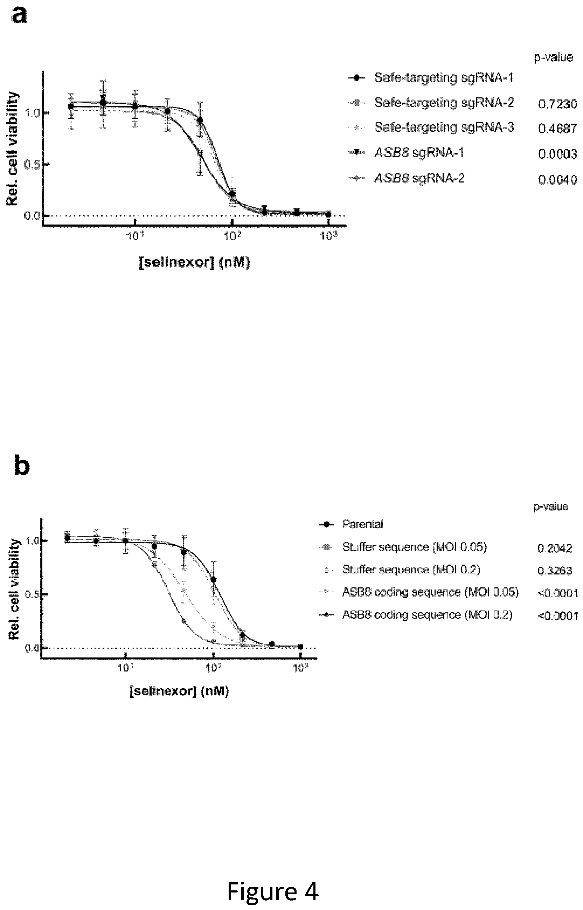

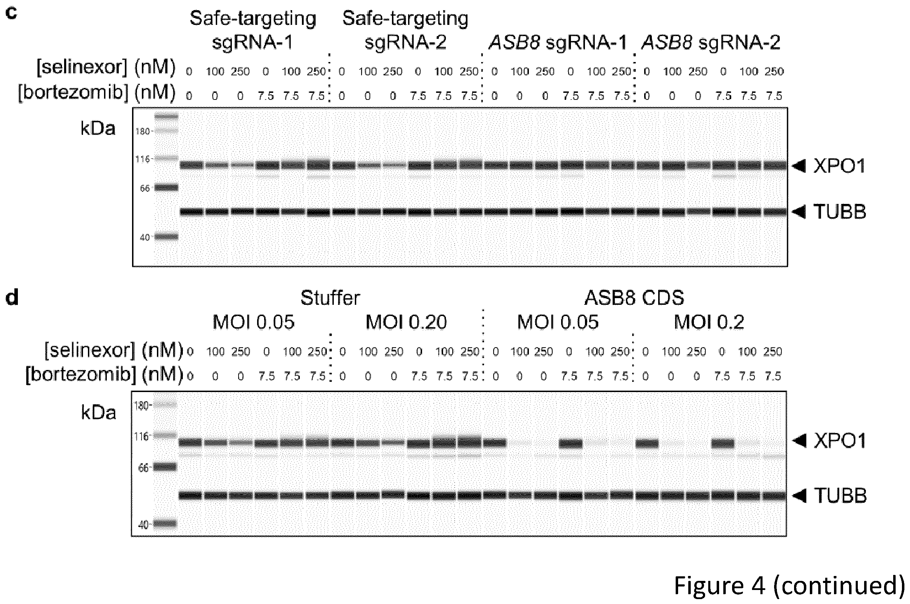

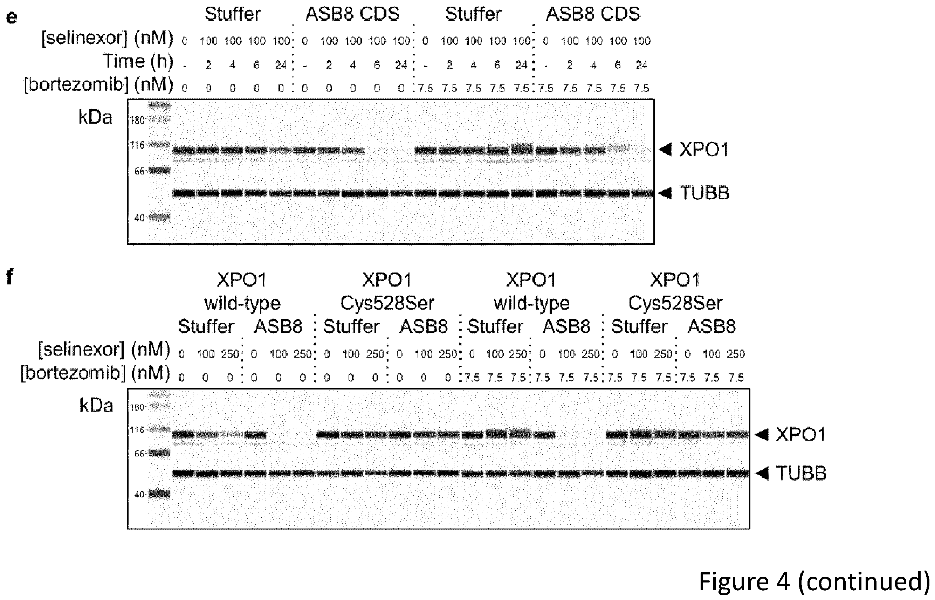

***Figure 4******.*** XPO1 protein degradation is regulated by ASB8. **a-b** Sensitization profile of HAP1 cells with either knockout or overexpression of *ASB8* under selection with selinexor. Cell viability when transduced with a safe-targeting or *ASB8* targeting sgRNA (**a**). Cell viability when transduced with a non-coding stuffer sequence or ASB8 coding sequence at two different multiplicities of infection (MOI) (**b**). After four days of treatment with compound at the indicated concentrations, cell viability was measured by MTS assay. Data were normalized to untreated cells and represent mean ±SD of three separate experiments performed in triplicate. IC₅₀ values were compared with control using an extra sum-of-squares F-test. **c-d** Monitoring of XPOL degradation in HAP1 cells with western blot. Comparison of cells stably transduced with a safe-targeting versus *ASB8* targeting sgRNA (**c**) or with a stuffer sequence versus ASB8 coding sequence (**d**). Cells were treated overnight with selinexor and bortezomib as indicated. Cells were harvested and total lysates were probed for XPO1 and TUBB (β-tubulin) protein levels. **e** Time-of-drug-addition assay in HAP1 cells harboring a stuffer or ASB8 coding sequence (MOI=0.2). Duration of treatment with selinexor is as indicated. For bortezomib, length of treatment was 24 h for all conditions. **f** Selinexor-induced degradation of XPO1 in HAP1 cells with an endogenous Cys528Ser mutation introduced by CRISPR-mediated homology-directed repair. Following stable transduction of XPOL wild-type and mutant cells with a stuffer or ASB8 coding sequence (MOI=0.2), cells were treated overnight with compound as indicated.

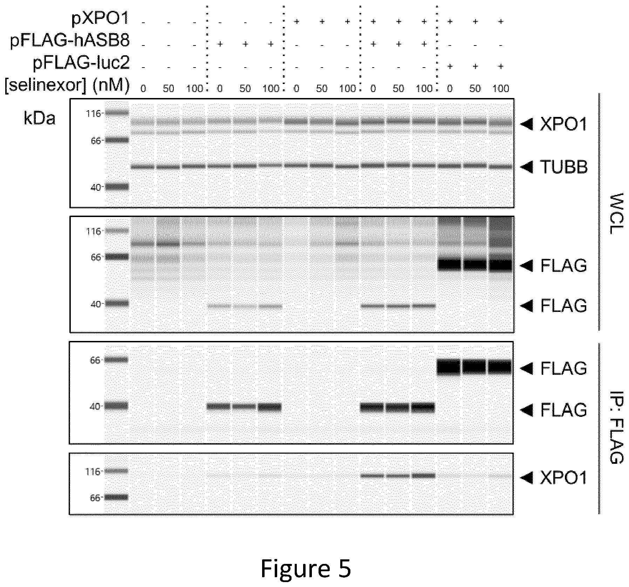

***Figure 5******.*** ASB8 interacts with XPO1. HEK293T cells were transiently transfected with XPO1 alone or together with either FLAG-ASB8 or FLAG-luc2 plasmids. After 24 hours, selinexor was added at the indicated concentrations and cells were incubated for 15 minutes. Next, cells were collected and incubated with lysis buffer for 20 minutes (whole-cell lysate, WCL). From these lysates, FLAG fusions were pulled down and co-immunoprecipitation of XPO1 was checked with western blot (IP: FLAG).

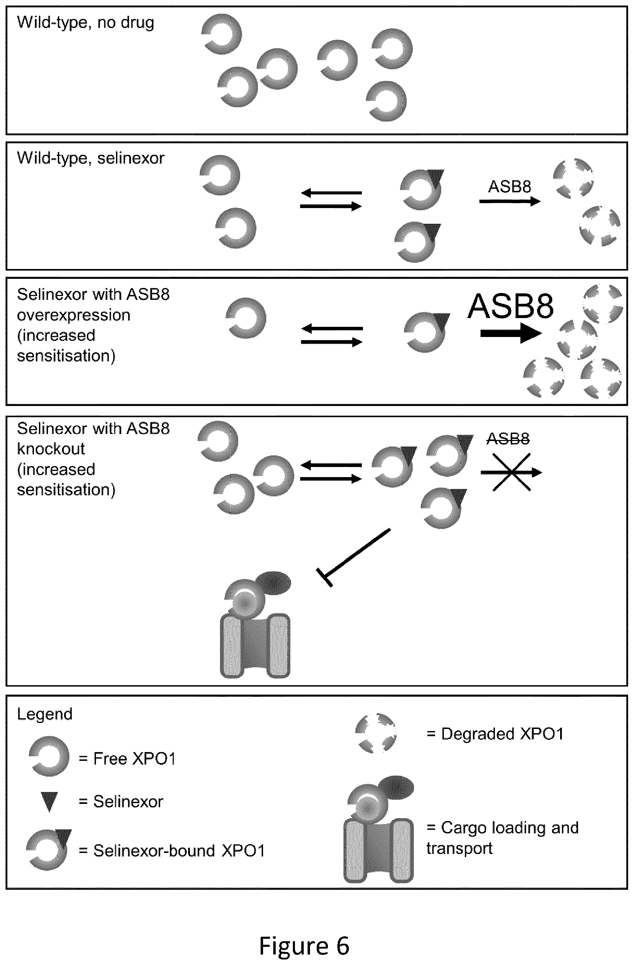

***Figure 6******.*** Model for the effect of ASB8 expression on selinexor sensitivity. Selinexor binds XPO1 in a dose-dependent manner implying that for a given concentration such as IC₅₀, a fixed fraction of the total XPOL amount will be occupied by inhibitor. ASB8 is part of the cellular machinery that promotes proteasomal degradation of selinexor-bound XPO1. In cells with endogenous ASB8 levels (wild-type), treatment with selinexor results in an overall decrease in XPOL protein concentration. When ASB8 is overexpressed, degradation is augmented leading to even less XPO1 protein. A lower concentration of free XPO1 denotes increased selinexor toxicity. Upon knockout of ASB8, selinexor-bound XPO1 is no longer degraded and the total XPO1 concentration is similar to the no-drug situation. Although the higher free XPO1 concentration is expected to bring about selinexor resistance, we instead observe sensitization. We speculate that the accumulated, inhibited XPO1 obstructs the nuclear export pathway by interfering with cargo loading and/or transport through the nuclear pore.

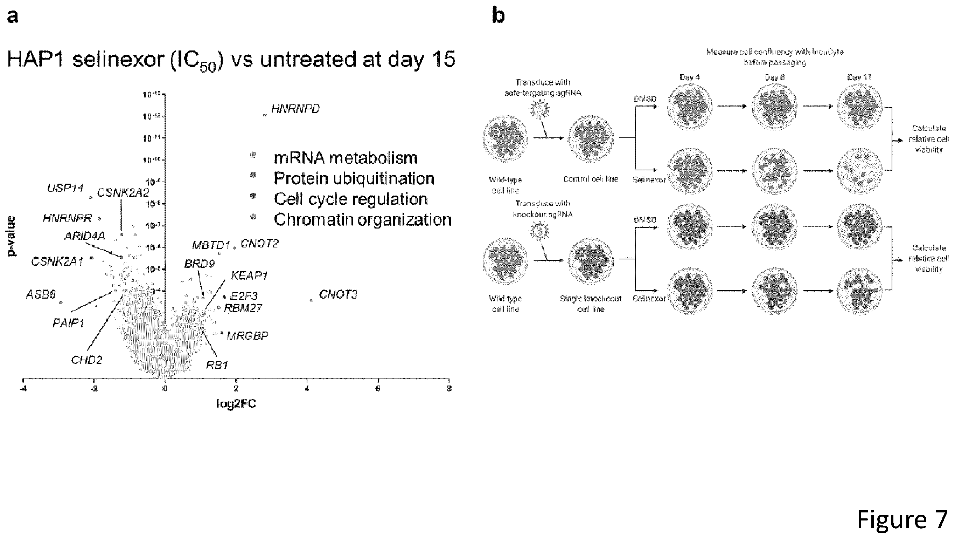

***Figure 7******.*** Genome-wide knockout screen in HAP1 (chronic myeloid leukemia) cells with selinexor and hit validation, **a** Gene-level comparison of drug-treated group versus control group after 15 days of selection with 100 nM selinexor (IC₅₀). Log₂(fold change) (log2FC) on the x axis represents the averaged difference of the four sgRNAs for each gene between the two groups. P-values on the y axis were calculated with CRISPRBetaBinomial⁵² (*See* **Methods**). **b** Setup of the cell confluency assay used for individual gene validation.

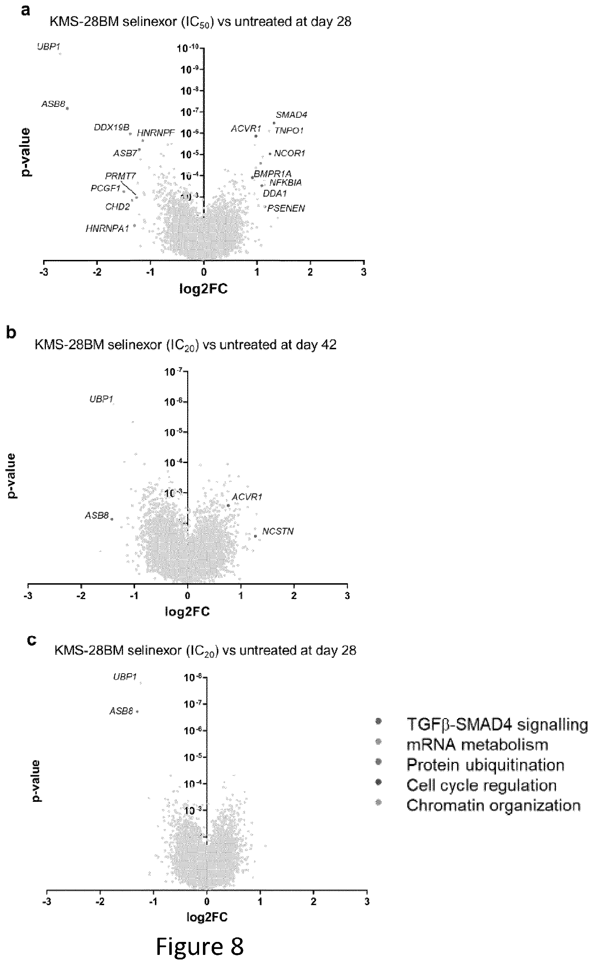

***Figure 8******.*** Genome-wide knockout screen with selinexor in KMS-28BM cells (multiple myeloma). **a-c** Gene-level comparison of drug-treated group versus control group in KMS-28BM cells at different time points and concentrations of selinexor: 28 days of selection at IC₅₀ (**a**), 42 days of selection at IC₂₀ (**b**) and 28 days of selection at IC₂₀ (**c**). Log₂(fold change) (log2FC) on the x axis represents the averaged difference of the four sgRNAs for each gene between the two groups. One-sided p-values on the y axis were calculated with CRISPRBetaBinomial⁵² (*See* **Methods**). **d** Assessment of on-target activity for a set of safe-targeting and gene-targeting sgRNAs by Sanger sequencing of the sgRNA target site in KMS-28BM cells transduced with a vector coding the indicated sgRNA and SpCas9. The 20-bp spacer sequence is indicated in blue, PAM sequence in red and cut site with a dashed line.

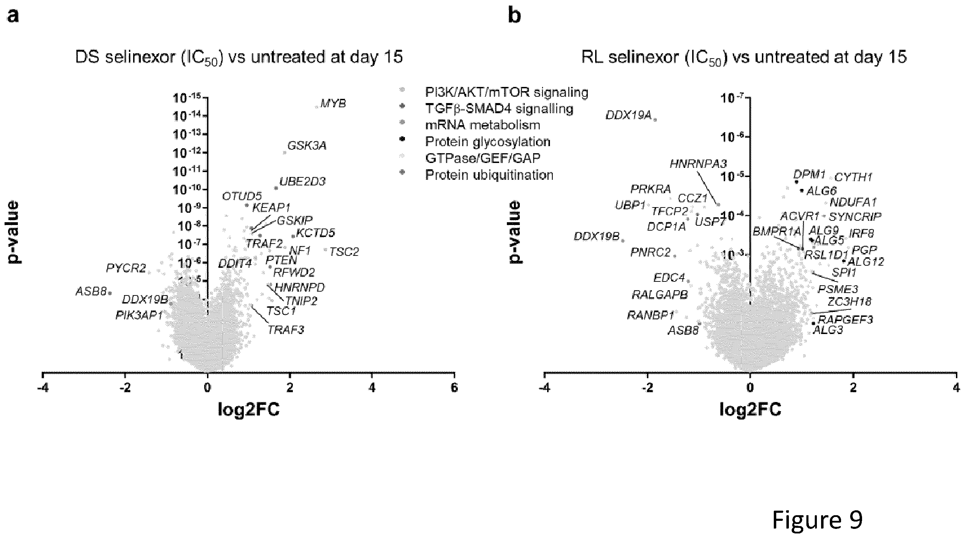

***Figure 9******.*** Genome-wide knockout screen with selinexor in DLBCL cell lines. **a-b** Gene-level comparison of drug-treated group versus control group after selection with selinexor (IC₅₀) at an intermediate time point (15 days) in DS (**a**) and RL cells (**b**). Log₂(fold change) (log2FC) on the x axis represents the averaged difference of the four sgRNAs for each gene between the two groups. One-sided p-values on the y axis were calculated with CRISPRBetaBinomial⁵² (*See* **Methods**).

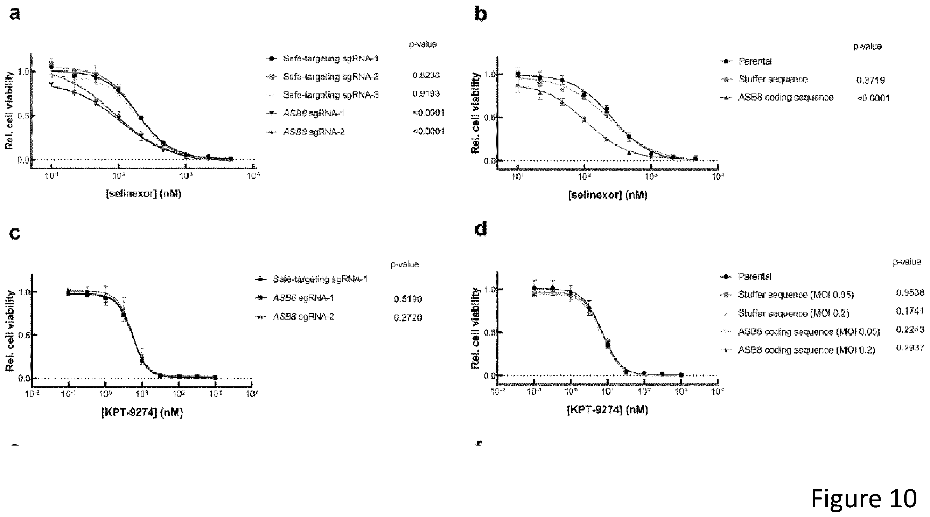

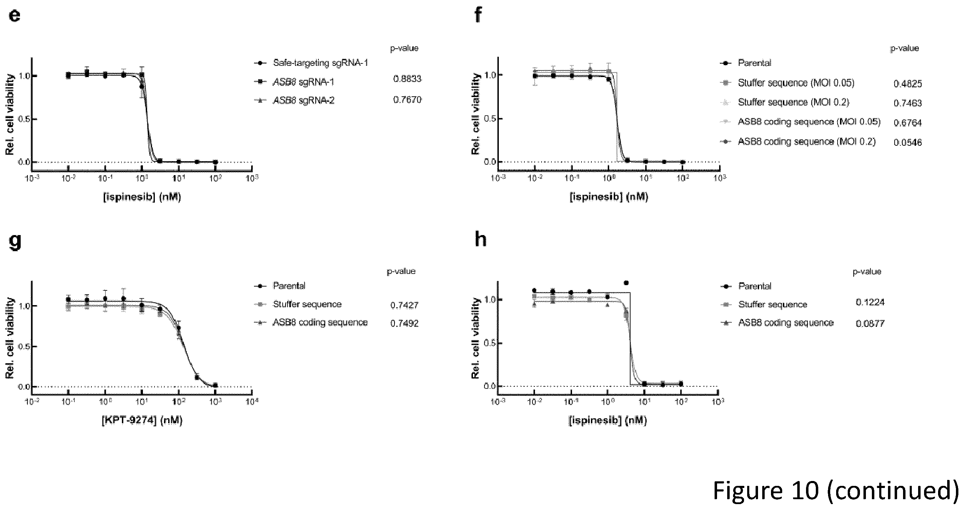

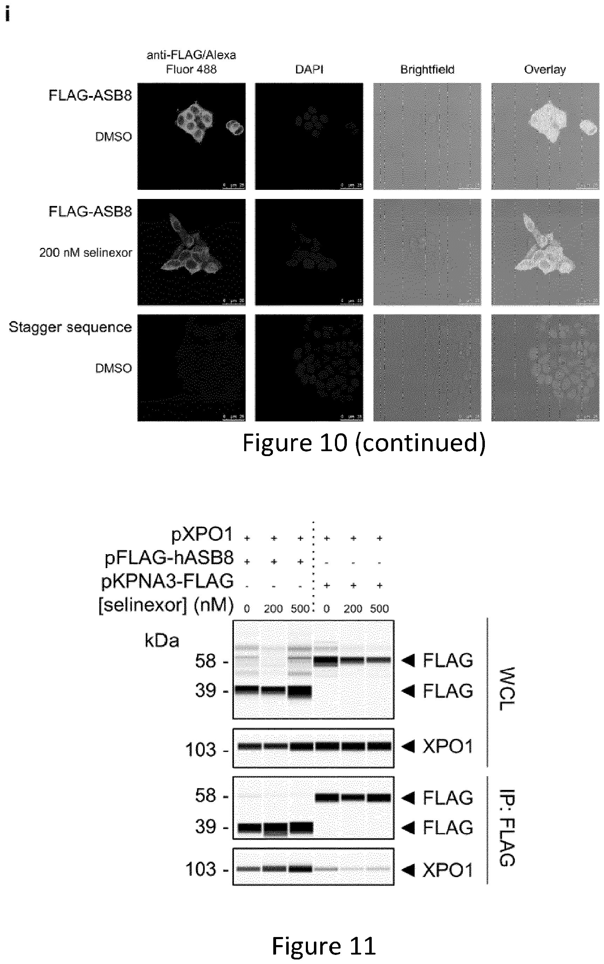

***Figure 10******.*** a Viability of KMS-28BM cells stably transduced with a stuffer or ASB8 coding lentiviral vector and treated with different concentrations of selinexor. **b-e** Viability of ASB8 knockout and ASB8 overexpressing HAP1 cells treated with different concentrations of KPT-9274 (**b**, **c**) or ispinesib (**d**, **e**). **f-g** Viability of ASB8 overexpressing KMS-28BM cells treated with different concentrations of KPT-9274 (**f**) or ispinesib (**g**). After four days (HAP1) or six days (KMS-28BM) of treatment with compound at the indicated concentrations, cell viability was measured by MTS assay. Data were normalized to untreated cells and represent mean ±SD of two or three separate experiments performed in triplicate. IC₅₀ values were compared with control using an extra sum-of-squares F-test. **h** Representative images of fluorescence microscopy imaging of subcellular ASB8 localization. HAP1 cells with a stably integrated FLAG-ASB8 or a non-coding stuffer sequence were treated overnight with DMSO or 200 nM selinexor. Cells were fixed and stained with a primary anti-FLAG antibody. Nuclei were stained with DAPI. ***Figure 11*****.** Co-immunoprecipitation of XPO1 with ASB8. HEK293T cells were transiently transfected with XPOL together with either FLAG-ASB8 or KPNA3-FLAG (control) plasmids. After 24 hours, selinexor was added at the indicated concentrations and cells were incubated for an additional 15 minutes. Next, cells were collected and incubated with lysis buffer for 20 minutes (whole-cell lysate, WCL). From these lysates, FLAG fusions were pulled down and co-immunoprecipitation of XPO1 was checked with western blot (IP: FLAG).

ASB8 (Ankyrin Repeat and SOCS Box containing 8) is a member of the ankyrin repeat and SOCS box (ASB) family of proteins which counts 18 members in humans and which function as the substrate recognition unit of an E3 ubiquitin ligase complex. Thus ASB proteins constitute the subunit of an E3 ubiquitin ligase complex responsible for recognition and binding to a specific target protein, initiating ubiquitination of the target and subsequent proteasomal degradation. The physiological targets of ASB8 and other ASB members remain largely unexplored.

The present invention demonstrates that ASB8 promotes selinexor-induced degradation of XPO1. In wild-type cells treated with increasing concentrations of selinexor, XPO1 will be degraded in a dose-dependent manner. In ASB8 knockout cells, XPO1 degradation is abrogated. However, in cells overexpressing ASB8, XPO1 degradation is more severe than in wild-type. Of note, degradation only happens in the presence of selinexor.

The finding that ASB8 in combination with a small molecule (selinexor) can trigger targeted protein degradation is a proof-of-concept that this system can be adapted to specifically target other proteins besides XPO1. Such small molecules only require binding to their target (which leads to degradation) without the need to also directly inhibit its function. This opens new possibilities to target proteins that were previously deemed undruggable. Moreover, using small molecules for targeted protein degradation could also have advantages over traditional occupancy-based inhibitors in terms of dosing, side effects and resistance mechanisms.

In the art is was difficult to devise methods to discover protein-degrading molecules because they need to fulfil two functions at the same time: As well as having affinity for their target, they also need to recruit the cellular degradation machinery (which can be any of an estimated 600 different E3 ligases). The observation that compounds such as selinexor (or other XPOL small-molecule binders) in combination with ASB8 is capable of performing is warrants further investigation into the capacity to degrade other targets in a similar fashion. The present invention discloses methods to identify compounds with therapeutic properties whose mechanism of action involves ASB -mediated, targeted protein degradation.

In the art three types of compounds are know that can induce protein degradation. Molecular glue

A molecular glue is a small molecule that acts as an adhesive by making two proteins, that normally would not interact, stick together. The molecular glue binds to one of the proteins, creating a new molecular surface that is now compatible with the surface of the second protein. In this case, both proteins and the small molecule all interact with each other.

### PROTAC (proteolysis targeting chimeras)

A PROTAC is a bifunctional molecule, a chimera consisting of two moieties connected via a linker. As each moiety binds to its own target, a PROTAC acts as bridge between two proteins. Typically, a PROTAC doesn't require direct interaction between the two proteins.

### Degron exposer

A degron exposer is a small molecule that binds its target, causing a conformational change or trapping the target in a specific conformation. This conformational change exposes a protein surface (degron) that is specifically recognized by a second protein (E3 ligase). In this case, there is no direct interaction between the small molecule and the second interacting protein (E3 ligase).

Compounds as determined by the methods of the present invention have drug-like properties capable of ablating a therapeutically relevant protein target by means of ASB8-mediated protein degradation. Different approaches and strategies might be used to achieve this goal.

### In vitro interaction assays

Herein methods can be used to search for compounds that enable binding of ASB8 to a predefined target or set of targets.

Examples hereof are co-immunoprecipitation assays to screen for compounds that facilitate ASB8 target binding.

Other examples are for example native mass spectrometry, FRET, HTRF, and thermal shift assay to study ASB8 interaction with other proteins in the presence of a compound.

Another method is proteomic profiling with promiscuous compounds such as 4-octyl itaconate. Itaconate and its derivative 4-octyl itaconate is a 'promiscuous' metabolite capable of modifying ~1000 protein targets including XPOL. 4-octyl itaconate modification might result in degron exposure in other targets besides XPO1.

In such profiling method a pair of isogenic cell lines are created with either ASB8 knockdown/knockout or ASB8 overexpression.

Cells are subsequently treated with 4-octylitaconate followed by mass spectrometry to determine protein abundances. Proteins depleted in the ASB8 overexpressing cells are potential targets of ASB8.

Hits of therapeutic relevance are used to design small-molecule degraders with increased specificity for that particular target.

Another method is involves a chemical screening

In cellular assay consisting of a pair of isogenic cell lines both cell lines are screened in parallel: one cell line with ASB8 knockdown/knockout and the other with ASB8 overexpression.

A library of small molecules or small-molecule fragments are screened in this assay to identify compounds that show a differential phenotype. Such compounds are indicative having a mechanism of action that involves ASB8.

The phenotyic read-out of such assay can be for example cell cytotoxicity, proliferation rate or apoptosis.

Typically a counterscreen is used to exclude modulators of XPO1.

Another methods involves a data mining approach

The methods rely on the use of available drug toxicity data to correlate drug toxicity with ASB8 expression in different cell lines. Compounds for which toxicity positively correlates with ASB8 expression are candidate ASB8-mediated degraders.

Hit confirmation is done by testing toxicity in ASB8 knockout and overexpression cells.

To detect gene loss-of-function events that modulate sensitivity to XPO1 inhibition, we conducted genome-wide CRISPR-Cas9 screens in cells under selection with selinexor. We selected HAP1 cells for our initial screen as this chronic myeloid leukemia cell line is a relevant model^{66,67} and has suitable characteristics for CRISPR screening, including a high proliferation rate and transduction efficiency. HAP1 cells were transduced with the Brunello genome-wide CRISPR knockout library in the one-vector configuration, in which sgRNA and Cas9 are expressed from a single lentiviral construct. Cells were split into control (DMSO) and selinexor-treated arms, further divided into three replicates per arm and passaged in parallel for 24 days (**Fig. 1a**). Selinexor was used at a concentration of 100 nM, corresponding to the IC₅₀, i.e., the concentration at which the live cell count in the treated arm was half of that in the control arm after three days. At each passage, cells were harvested and per arm, an equal number of cells was reseeded with fresh compound. We reasoned that selecting at IC₅₀ was adequate to enrich knockouts that confer resistance while at the same time still detect the bulk of sgRNAs, to allow relative depletion of sensitizing knockouts. From day 22 onwards, treated cell populations were becoming noticeably resistant, indicating that screening duration had been sufficient.

Cells were harvested, genomic DNA extracted and sgRNA cassettes were amplified with nested PCR using primers harboring Illumina adapter and barcodes sequences. Next-generation sequencing was used to quantify sgRNA abundances in each sample. sgRNA-level and gene-level comparisons were performed with the CRISPRBetaBinomial algorithm on samples collected at an intermediate (day 15) and at the final time point (day 24) (**Fig. 1b**, **Fig. 7a**)⁵². Overall, there was good agreement between both time points though absolute Log₂(fold change) (log2FC) values were more pronounced at the later time point as a result of prolonged enrichment or depletion.

We used the Uniprot and Reactome databases in combination with existing literature to examine molecular functions and pathway annotations for the top hits^{68,69}. Among the top hits is a remarkable group of genes involved in mRNA processing and turnover. Notably, *CNOT2* and *CNOT3* are members of the multi-subunit CCR4-NOT complex which is responsible for targeted mRNA decay through deadenylation⁷⁰. In a similar fashion, *HNRNPD* (*AUF1*) controls mRNA stability through binding of AU-rich elements (AREs) found in the 3' UTRs of many mRNA species⁷¹. Interestingly, different members of the same biological process are either sensitizing or buffering upon knockout. While knockout of *HNRNPD* confers resistance to selinexor, another member of the HNRNP family, *HNRNPR,* appears to have an antagonistic function as knockout of this gene has a sensitizing effect^{72,73}. Likewise, we identify knockout of the poly(A)-binding protein-interacting protein 1 (*PAIP1*) as sensitizing while RNA-binding protein 27 (*RBM27*) is buffering.

A second group of hits consists of genes involved in protein degradation through ubiquitination, including *ASB8, USP14* and *KEAP1.* Thirdly, we identify the retinoblastoma (*RB1*) tumor suppressor, which has a central role in cell cycle regulation and is a validated cargo of XPO1⁷⁴. Independent confirmation of this hit comes from the identification of the RB1-modulating genes *E2F3* and *ARID4,* that also emerge from this screen. Fourthly, we distinguish a group of genes involved in chromatin remodeling: *BRD9, MRGBP* and *CHD2.*

For validation of the hits, we first sorted genes according to log2FC and selected the top 13 for both positive and negative log2FC values and p-value <0.01. For each gene, we designed two new knockout sgRNAs by selecting the top two picks from the Gene Perturbation Platform that were not already part of the Brunello library⁶. For control guides, we selected three safe-targeting sgRNAs with a target sequence located in a non-functional, non-genic region⁵³. Knockout and safe-targeting sgRNAs were used to construct single-knockout and control HAP1 cell lines. For the purpose of validation, we employed a cell confluency assay to monitor relative growth inhibition in selinexor-treated knockout cells compared to a safe-targeting control (**Fig. 7b**). The majority of these top-ranking genes was validated (**Fig. 1c**) though overall levels of sensitization or resistance were moderate.

### The TGFβ-SMAD4 pathway is central to selinexor resistance in multiple myeloma

In the next step, we shifted our focus to multiple myeloma since this is the first and most significant cancer type for which selinexor has obtained FDA approval at the time of writing. Identifying modulators of sensitivity in this cancer type could prove useful in interpreting or predicting patient response when coupled with patient genomic sequencing and gene expression data. We therefore conducted screens in KMS-28BM and SK-MM1 multiple myeloma cells following the same protocol as for HAP1 cells but extending the screen duration, consistent with the longer doubling time of each cell line (**Figs. 2a****, b**)**.** For the slower growing KMS-28BM cells-which have a doubling time of 38 hours-we found that a prolonged screen allows continued enrichment of resistance conferring knockouts. When comparing time points at day 28 with day 42, log2FC values and distribution of depleted genes are very similar, while for enriched genes, log2FC are more distinct at the later time point (**Fig. 2a****,** **Fig. 8a**). Besides treating cells with IC₅₀, we also maintained a treatment arm with IC₂₀ as we reasoned that a sub-lethal dose might be better suited to maintain cell viability over a long time course and should only reveal interactions with the strongest phenotype (**Fig. 8b****, c**). Indeed, results from this arm recapitulate the two strongest sensitizer genes, *UBP1* and *ASB8,* but the lower concentration insufficiently modulates XPO1 as it fails to isolate most other hits found in the IC₅₀ arm, especially resistance conveying knockouts.

The TGFβ-SMAD4 pathway emerges as the main pathway involved in selinexor resistance (**Figs. 2a****, b**). *SMAD4,* a transcription factor with tumor suppressor function and the central mediator of this pathway, is one of the main hits in both cell lines. Furthermore, *BMPR1A* and *ACVR1* are members of the bone morphogenetic protein type I receptors, a family of transmembrane proteins that bind members of the TGFβ superfamily of ligands and which function upstream of *SMAD4⁷⁵. PSENEN* and *NCSTN* are subunits of the gamma-secretase complex, responsible for cleaving the transmembrane domain of NOTCH and subsequent release of its intracellular domain. Crosstalk between the Notch and TGFβ signaling pathways has been shown to occur at the level of the NOTCH intracellular domain, that is capable of interacting with SMAD3, further promoting SMAD3 and SMAD4 complex formation^{76,77}. Finally, *NCOR1* plays a role in balancing TGFβ signaling activity. NCOR1 is recruited to the DNA-bound SMAD4 complex by SKI and SKIL and presumably antagonizes its transcription factor activity⁷⁸. For the SK-MM-1 cells, besides *SMAD4,* we identify *GATA6,* a transcription factor that binds BMP response element DNA sequences thus stimulating autocrine TGFβ signaling, including phosphorylation of SMAD2/3⁷⁹, and TGFB3, a cytokine belonging to the TGFβ superfamily.

Another common hit in both multiple myeloma cell lines is *NFKBIA,* the well-known inhibitor of NF-κB and also cargo of XPO1, whose knockout has previously been demonstrated to render cells resistant to selinexor⁸⁰⁻⁸⁵. In addition, we identify common synthetic lethal interactions with *UBP1* knockout, a member of the *TFCP2* family of transcription factors⁸⁶, and *ASB8* knockout, a putative substrate-recognition domain of a SCF-like Elongin-cullin-SOCS (ECS) E3 ubiquitin-protein ligase complex. Just like in HAP1, the KMS-28BM screen delivers hits involved in mRNA binding and processing, including members of the heterogeneous nuclear ribonucleoproteins family (*SYNCRIP, HNRNPF* and *HNRNPA1*)*, DDX19B* and *SCNM1.* To get a better understanding of the levels of resistance/sensitization, we selected a set of four hits and validated them individually (**Fig. 2c****)**. We used MTS cell viability assays to establish selinexor dose-response curves in single knockout cell lines. Although the effect of knockout on selinexor sensitivity was small, IC₅₀ values differed significantly from control cells, indicating that our CRISPR screens managed to pick up subtle changes. We checked editing of the target loci with Sanger sequencing and observed disrupted DNA sequences for each sgRNA target locus except for *SMAD4* sgRNA-2, which was only partially edited, and safe-targeting sgRNA-3, which remained unedited . The latter is probably due to poor on-target activity, so sgRNA-3 should be considered as non-targeting rather than safe-targeting.

Next, we compared our CRISPR screen data to patient gene expression and survival data. We analyzed RNA-seq data from 73 patients enrolled in the Phase 2b STORM (Selinexor Treatment of Refractory Myeloma) clinical trial who underwent treatment with selinexor and dexamethasone. These patient data were used to interrogate the Reactome gene sets within the Molecular signatures database (MSigDB)⁵⁸⁻⁶⁰. For each patient, we used gene expression data to calculate a gene set enrichment analysis score for each of the approximately 1500 curated pathways in this database. We then correlated these scores with progression free survival (PFS) by running a Cox proportional hazards model. Within the top ten most predictive pathways in terms of PFS, we found four closely related gene sets within the TGFβ signaling pathway (**Fig. 2d**). Moreover, "TGFβ receptor signaling activates SMADs" was the most statistically significant pathway for which higher activity levels were associated with longer PFS (**Fig. 2e**). The observation that upregulation of these pathways is associated with prolonged PFS is in agreement with our CRISPR screen data as the latter reveal that knockout (i.e., downregulation) of several genes within the broader "Signaling by TGFβ family members" group render cells more resistant to selinexor. These data suggest that expression levels of a subset of genes in the TGFβ signaling pathway are predictive of clinical benefit following treatment with selinexor in patients with multiple myeloma.

### Screens in DLBCL further highlight inter-cell-line diversity and the importance of genetic background

Results thus far indicate that genetic interactions with selinexor are highly cell- and cancer-type-specific. Therefore, to better grasp this diversity, we expanded the scope of our screens by including diffuse large B-cell lymphoma, as this is the second FDA-approved cancer indication for selinexor²⁸. We selected two suitable DLBCL cell lines, DS and RL, and subjected them to CRISPR knockout screening with selinexor at IC₅₀ as before (**Fig. 3a**, **b**, **Fig.9a****, b**). Lentiviral transduction proved to be less efficient in these cell lines; therefore, instead of the single-vector library, we switched to using the smaller lentiGuide-Puro backbone, encoding the sgRNA but not SpCas9. This library was used in conjunction with DS and RL cells stably expressing SpCas9.

Results from DS and RL cells show partial overlap with earlier screens. In particular, knockout of *ASB8* consistently emerges as a sensitizer in all five cell lines. In DS cells, we find the mRNA regulating genes *DDX19B* (sensitizing, s) and *HNRNPD* (resistance conferring, r)while in RL cells, we identify *DDX19A* (s), *DDX19B* (s) and *SYNCRIP* (*HNRNPQ*) (r). Similar to multiple myeloma cells, the TGFβ-SMAD4 is of importance in RL cells (*BMPR1A, ACVR1, SMAD4* and *SMAD5* (r)) but apparently not in DS cells. In addition, knockout of *UBP1* sensitizes RL cells to selinexor, as does knockout of the closely related *TFCP2* transcription factor⁸⁶, independently validating this result.

Most hits however are unique to either cell line. In DS cells, knockout of a series of negative regulators of PI3K/AKT/mTOR signaling conveys resistance to selinexor (*GSK3A, TSC1*/*2, PTEN, NF1* and *DDIT4*) while knockout of *PIK3AP1* and *FIG4* convey sensitization^{87,88}. These results are similar to an earlier report that employed CRISPR screening with selinexor in acute myeloid leukemia cells and that demonstrated that selinexor treatment leads to upregulation of PI3K/AKT singaling¹⁵. Furthermore, we find genes involved in protein ubiquitination (*KCTD5* (r), *UBE2D3* (r), *RFWD2* (r), *TRAF2*/*3* (r), *UBAP2L* (r), *USP34* (s), *UBE2E1* (s),...) but these are functionally very diverse with various protein targets. Of note, *TRAF2*/*3* and *TNIP2* have been implicated in NF-εcB activation in B cells suggesting a similar mechanism as seen with knockout of *NFKBIA* in multiple myeloma^{89,90}. Results from the RL cell line add yet another dimension to the collection of response-modifying genes. Hits from this screen include genes involved in GTPase regulator activity (*CYTH1* (r), *RAPGEF3* (r), *ARL2* (r), *CCZ1* (s) and *RALGAPB* (s)) and genes involved in oligosaccharide biosynthesis/protein glycosylation (*ALG12* , *ALG9* , *ALG3* , *DPM2 , DPM1* , *ALG6* and *ALG5* (r)).

A comparison of hits across all cell lines, timepoints and drug concentrations confirms the considerable inter-cell-line variability, but at the same time highlights some remarkable similarities between different models ). Regarding resistance knockouts, the top three genes in mRNA processing (*CNOT3, HNRNPD* and *CNOT2*) found in HAP1 cells (CML) are recapitulated in DS and/or RL cells (DLBCL). Furthermore, there is overlap between KMS-28BM and RL cells, most notably *SMAD4, BMPR1A* and *ACVR1.* As for sensitizers, *ASB8* is the only universal hit recurring in all cell lines and screen conditions. *UBP1* is another nearly universal sensitizer with the exception of DS cells, in which *UBP1* knockout shows the opposite effect (resistance). This pattern is highly similar to the one observed for the related gene *TFCP2,* which together with *UBP1* belongs to the TFCP2/Grainyhead family of transcription factors. Additionally, *DDX198* appears in four out of five cell lines. This is further confirmed by earlier reports stating that lower expression of this gene in cell lines correlates with drug sensitivity against KPT-276, an analog of selinexor⁹¹.

. Furthermore, dissimilar results from the KMS-28BM cell line screened at two different concentrations emphasize the importance of drug concentration. Selective pressure at IC₂₀ was inadequate compared to IC₅₀ to separate modulators of resistance and sensitization from background variation.

### Penetrant synthetic lethal interaction between XPO1 inhibition with selinexor and ASB8 knockout

Since *ASB8* was the only gene that universally modified response to selinexor in all tested cell lines, we focused on elucidating the molecular pathways underlying this drug-gene interaction. *ASB8* belongs to the ankyrin repeat and SOCS box (ASB) family of proteins that function as the substrate recognition subunit in a subset of ECS E3 ubiquitin ligases. The physiological targets of ASB proteins remain largely unknown. ASB8 for instance has been linked to innate immune response following infection with porcine reproductive and respiratory syndrome virus with IKKβ and TBK1/IKKε among its proposed targets^{92,93}.

We first checked sensitivity to selinexor in ASB8 knockout and overexpressing cells, expecting to find opposite effects. Remarkably, both knockout and overexpression convey increased sensitivity to the drug, with the effect being more pronounced in ASB8 overexpressing cells (**Fig. 4a**, **b**, **Fig. 10a****, b**)**.** Next, to exclude that sensitization is unspecific, we determined dose-response with two random, targeted cancer drugs: KPT-9274, an inhibitor of NAMPT²², and ispinesib, an inhibitor of KIF11⁹⁴. For these two compounds, there was no difference in response between control and ASB8 knockout/overexpressing cells (**Fig**. **10c****-h**)**.**

ASB8 has not been reported to be a cargo of XPO1, though computational tools predict one (Wregex) or three (locNES) candidate NESs, all situated at the C-terminus of the protein^{95,96}. However, in stable FLAG-ASB8 expressing HAP1 cells the protein is mainly located in the cytoplasm and we did not observe nuclear accumulation of ASB8 upon treatment with selinexor, indicating that ASB8 is not a cargo of XPO1 (**Fig. 10i**).

### ASB8 mediates selinexor-induced proteasomal degradation of XPO1

SINE compounds have been previously reported to reduce XPOL protein levels upon binding^{23,97}. While the precise mechanism is unclear, it likely involves proteasomal degradation since the effect is abrogated by addition of the proteasome inhibitor bortezomib. We reasoned that since ASB8 presumably targets proteins for ubiquitin-mediated degradation, it might be involved in regulating XPO1 protein turnover itself. While knockout or overexpression of ASB8 alone did not alter XPO1 protein levels, there was a distinctive effect upon addition of selinexor. In control cells harboring a safe-targeting sgRNA, overnight treatment with selinexor causes dose-dependent degradation of XPO1, which was prevented by treatment with bortezomib (**Fig. 4c**). However, we did not observe selinexor-induced XPOL degradation to the same extent in ASB8 knockout cells. Moreover, in ASB8 overexpressing cells, XPO1 was profoundly depleted even in the presence of bortezomib (**Fig. 4d**). In a time course experiment, XPO1 was degraded more rapidly in ASB8 overexpressing cells compared to control and this degradation was somewhat delayed by bortezomib (**Fig. 4e**). Taken together, with regard to XPO1 degradation, ASB8 knockout and overexpression showed opposite phenotypes, both of which apparently sensitize cells to selinexor. To investigate whether ASB8-mediated XPOL degradation is specifically dependent on selinexor, we repeated the degradation assay in cells harboring an endogenous *XPO1* Cys528Ser mutation, the central residue in the NES-binding cleft to which selinexor covalently binds. In these mutant cells, selinexor did not trigger XPO1 degradation, neither in control nor ASB8 overexpressing cells (**Fig. 4f**), demonstrating that binding of selinexor to XPO1 is specifically required for ASB8-mediated degradation.

Next, we sought to investigate whether ASB8 physically interacts with XPO1. We performed a pull-down experiment where XPO1 was co-transfected with FLAG-ASB8 in HEK293T cells. We used FLAG-luc2 or KPNA3-FLAG as negative controls. Before lysis, cells were treated briefly with selinexor for 15 minutes to allow cellular uptake of the drug but short enough to forestall degradation of XPO1. Selinexor was also added to lysis and wash buffers at the indicated concentrations. Upon pull-down with anti-FLAG magnetic beads, XPOL co-immunoprecipitated with ASB8 but not luc2 or KPNA3 (**Fig. 5****,** **Fig. 11**). Moreover, adding higher concentrations of selinexor increased the amount of immunoprecipitated XPO1(**Fig. 11**), suggesting that ASB8 directly or indirectly interacts with XPO1 and that this interaction is facilitated by selinexor.

Pharmacologic inhibition of XPO1 has emerged as an exciting therapeutic strategy to treat a wide variety of hematologic and solid malignancies. For this targeted therapy to reach its full potential, optimal drug combination regimens and predictive biomarkers need to be established, but these require profound insight into the genetic drivers of drug effectiveness and resistance. Genome-scale CRISPR-Cas knockout screening offers an excellent tool to study drug-gene interactions. We performed such screens with selinexor, a clinically approved, orally bioavailable, small-molecule inhibitor of XPO1, and identified a diverse set of genes that modulate drug sensitivity.

A number of hits identified in our screens corroborate selinexor's mechanism of action i.e., reestablishment of tumor suppressor activity by means of nuclear entrapment, which also verifies the validity of the screens. For example, NFKBIA (IκB-α), an inhibitor of the NF-εcB complex, is a prototype cargo of XPO1⁸⁰⁻⁸². Selinexor prevents nuclear export and subsequential proteasomal degradation of IκB-α, thus enabling it to resume its inhibitory function resulting in downregulation of NF-εcB pro-tumoral transcriptional activity. Silencing of IκB-α disrupts this sequence of events which causes selinexor to loose part of its potency, explaining why this gene was identified in our screens⁸³⁻⁸⁵. A similar mechanism of resistance explains the identification of SMAD4, another tumor suppressor and cargo of XPO1⁹⁸. Moreover, we identified several members involved in TGFβ-SMAD4 signaling that cause resistance upon knockout (*BMPR1A, ACVR1, SMAD5, PSENEN,* *NCSTN, NCOR1* and *GATA6*), underscoring the importance of this pathway in selinexor's mechanism of action.

We reanalyzed gene expression data from patients prior to receiving treatment with selinexor, with a focus on pathway activity levels rather than expression levels of individual genes. In agreement with our CRISPR screens, we found that the pathway "TGFβ receptor signaling activates SMADs" within the Reactome database strongly correlates with PFS. CRISPR hits such as *ACVR1* and *BMPR1A* are part of the related Reactome "Signaling by BMP" pathway rather than the "TGFβ receptor signaling activates SMADs" but both pathways belong to the wider "Signaling by TGFB family members" group. These have in common that their activation results in mobilization of SMAD proteins with SMAD4 as a central node. It is reasonable to assume that pathway activity is a better predictor of response to a drug rather than the expression status of a single or a few genes. The results from this study warrant further investigation into the capability of using patient gene expression level data from TGFβ pathway members to predict clinical response to selinexor. This could ultimately lead to the rational assembly of an optimal set of biomarkers with strong predictive power.

For many other hits, the mechanism of resistance or sensitization remains unclear. A remarkable observation is the large number of genes involved in mRNA metabolism e.g., *DDX19B, DDX19A, HNRNPA1, HNRNPF, SYNCRIP, CNOT2, CNOT3, HNRNPR, HNRNPD,...* These proteins have diverse functions and regulate gene expression of their targets through RNA transcription, pre-mRNA splicing, mRNA degradation/stabilization, mRNA nuclear export and translation. Most have confirmed or presumed nucleocytoplasmic shuttling activity and can act as adaptor molecules in XPO1-mediated mRNA nuclear export^{95,99,100}. Through their roles in mRNA metabolism, these proteins regulate expression of genes involved in tumorigenesis such as *MYC, SRC, FOS* and *BCLX.* Moreover, the equilibrium of these RNA-binding proteins is finely regulated and differential expression and localization is frequently observed in cancer¹⁰⁰⁻¹⁰³. For instance, mutations in *CNOT3,* a member of the CCR4-NOT deadenylase complex, have been found to be associated with T-cell acute lymphoblastic leukemia¹⁰⁴. Further study established that *CNOT3* functions as a tumor suppressor with knockdown resulting in increased tumor formation. These effects were attributed to mRNA stabilization and subsequent overexpression of *CCN81* (Cyclin B1) and other genes involved in DNA replication and ribosome biogenesis¹⁰⁵. It is conceivable that selinexor entraps CNOT3 in the nucleus, leading to altered deadenylase activity and downregulation of its mRNA targets involved in tumorigenesis. Upon knockout of CNOT3, expression of target genes is no longer kept in check and cells lose sensitivity to selinexor. Yet another study has reported that selinexor alters nycleocytoplasmic shuttling of hnRNP A1 in Ph⁺ chronic myelogenous leukemia resulting in downregulation of the *SET* oncogene⁶⁶. In conclusion, our data suggest that nuclear export inhibition of mRNA-binding proteins makes a substantial but largely overlooked contribution to selinexor's antitumoral effect.

As a general observation from our screens, knockout of a single gene confers only a mild level of resistance or sensitization to selinexor. This reflects XPO1's central role in cellular homeostasis with >200 characterized and potentially >1000 export targets^{99,106}. Other interaction partners such as STK38, which mediates phosphorylation of XPO1, indicate that XPO1 function is tightly regulated, further adding to XPO1's extensive interaction netwerk⁶⁴. Since multiple proteins and pathways contribute to selinexor's antitumoral affect, it is highly reasonable that a single gene disruption can only trigger a mild effect. Moreover, XPO1 itself does not emerge from knockout screens since it is a core essential gene and knockouts are quickly lost from both control and treated populations^{91,107}. This sets selinexor apart from screens with compounds with a non-essential target, where silencing of a single gene, typically the drug target, leads to prominent resistance^{108,109}. Performing multiplex screens will be an interesting option to explore to what extent combined knockout of two or more targets evokes additive effects and also to uncover previously overlooked modulators of resistance masked by the existence of paralogous genes with redundant function^{110,111}.

SINE not only block docking of cargo molecules onto XPO1, they also reduce XPOL protein levels through proteasomal degradation, though the precise mechanism remains unclear^{23,97}. Our screens demonstrate that knockout of ASB8 is synthetically lethal with XPOL inhibition in all screened cell lines and that this protein triggers selinexor-induced proteasomal degradation of XPO1. Interestingly, both overexpression and knockout sensitize cells to selinexor. In treated cells, selinexor occupies a fraction of the cellular XPO1 pool in a dose-dependent manner (**Fig. 6**)¹¹². Selinexor toxicity is inversely proportional to the remaining concentration of free, uninhibited XPO1 protein. In wild-type cells, ASB8 mediates removal of non-functional, selinexor-bound XPO1. Degradation is somewhat balanced out by increased XPO1 expression in an attempt to maintain XPO1 functional activity²³, though total XPOL protein levels are lower than in the no-drug condition. In cells overexpressing ASB8, we propose that degradation is further increased, leading to dramatic XPO1 depletion and heightened drug sensitivity. On the other hand, ASB8 loss-of-function abrogates degradation and leads to accumulation of inhibited XPO1. Because the total XPO1 concentration is similar to the no-drug situation, the equilibrium of selinexor occupancy implicates concurrent accumulation of free XPO1 and consequently, an expected increase in selinexor resistance. This however conflicts with our observation that ASB8 knockout triggers sensitization. One explanation for this apparent paradox might be that while inhibited XPO1 isn't capable of binding or transporting cargo, it might hinder nuclear export by interacting with other factors of the XPO1-mediated export pathway, again culminating in increased drug sensitivity. All taken together, we propose that endogenous ASB8 mitigates selinexor's toxicity by mediating controlled degradation of inhibited XPO1. Aberrant levels of ASB8, either overexpression or knockout, cause a disturbance in equilibrium both resulting in increased drug sensitivity. In this respect, knockout of ASB8 resembles treatment with bortezomib, whose complete inhibition of the proteasome also abrogates SINE-induced degradation of XPO1 and which often acts synergistically with selinexor. In addition to ASB8, knockout of ASB7 also showed sensitization in one CRISPR screen (KMS-28BM). It is conceivable that this ASB family member has a similar function as ASB8.

It is not clear how exactly ASB8 recognizes selinexor-bound XPO1 and what its biological relevance might be. Small SINE compounds like selinexor only partially occupy the XPO1 cargo-binding cleft, forcing part of its hydrophobic surface to remain exposed. When these exposed hydrophobic pockets are shielded by SINE compounds with longer substituents or become disrupted by mutagenesis, drug-induced degradation is blocked¹¹³. This suggests that selinexor itself together with the exposed, hydrophobic cleft surface constitute a degron that is directly or indirectly recognized by ASB8. The ability of SINE compounds, unlike leptomycin B, to induce XPOL degradation might be one of the reasons for their superior tolerability in normal cells. Not only might this pathway present an alternative strategy to regulate cellular XPOL levels and activity, it could also be adapted to modulate other ubiquitin ligase-substrate interactions. Regarding the biological function of ASB8, its capacity to dispose of non-functional XPO1 is likely part of the protein quality control machinery. In a similar fashion, covalent binding of selinexor might mimic an endogenous, posttranslational modification of the XPOL C528 target residue (e.g., S-nitrosylation¹¹⁴) that triggers proteasomal degradation.

In conclusion, our CRISPR loss-of-function screens with selinexor uncover diverse drug-gene interactions, illustrating both known and novel aspects of XPOL-mediated nuclear export inhibition. As selinexor will become more widely adopted as a therapy for multiple myeloma and other cancer indications, we can expect more genomic sequencing and gene expression data from patients will be generated. Besides the need for sufficient patient data, such data typically originate from heterogenous patient populations with diverse phenotypical characteristics and medical histories. Also, stratification of responders and non-responders based on different parameters such as tumor size, progression-free survival, overall survival or other physiological parameters can be ambiguous and is further confounded as selinexor is used not as a single agent but rather in combination with other drugs. Data from our *in vitro* chemogenetic screens offer a framework to assist in interpreting patient sequencing data and aid in discriminating noise from meaningful factors with true biomarker potential. These data add valuable insight into the mechanisms of resistance and sensitization in play. However, to capture the breadth of genomic alterations underlying cancer development and drug response/resistance, screening additional cancer models will most likely be required.

### EXAMPLES

### Example 1. Cell Culture and compounds

HAP1 cells were obtained from Horizon Discovery. KMS-28BM cells were ordered at the JCRB Cell Bank. SK-MM-1 cells were from Vienna, Austria. DS, RL and HEK293T cells were obtained from ATCC. HAP1 cells were cultured in IMDM (Gibco) with 10% fetal bovine serum (FBS) (Biowest). KMS-28BM and DS cells were cultured in RPMI 1640 (Gibco) with 20% FBS. SK-MM-1 and RL cells were cultured in RPMI 1640 (Gibco) and 10% FBS. HEK293T Cells were grown in DMEM (Gibco) and 10% heat-inactivated FBS. 20 µg/mL gentamicin was added to all media. Cells were cultured in humidified incubators at 37 °C and 5% CO₂.

KPT-330 (selinexor) and KPT-9274 were provided by Karyopharm Therapeutics (Newton, MA). Ispinesib and bortezomib were purchased from SelleckChem. Q-VD-OPh was ordered from MedChemExpress. Compounds were dissolved in dimethyl sulfoxide (DMSO).

### Example 2. CRISPR-Cas9 library amplification

The plasmid pools from the human Brunello knockout library in plentiCRISPRv2 and plentiGuide-Puro were obtained from Addgene (#73179, #73178)⁶. Plasmid amplification and lentiviral vector production were as previously described⁵¹. Briefly, plasmid DNA was electroporated into Endura ElectroCompetent cells (Lucigen) in six parallel reactions with 100 ng DNA per 25 µL bacteria. Transformed bacteria were plated onto 15-cm agar plates with 100 µg/mL ampicillin and incubated at 32 °C for up to 18 h. Library coverage was calculated to be >9,000-fold by counting colonies from a set of serial dilution plates. Colonies were scraped off and plasmid DNA was purified with the PureLink HiPure plasmid maxiprep kit (Invitrogen).

Lentivector was produced by seeding 35 150-cm² flasks with 9 × 10⁶ HEK293T cells per flask in 20 mL of DMEM supplemented with 10% heat-inactivated FBS. The next day, library plasmid DNA (8 µg per flask) was cotransfected with psPAX2 (4,8 µg) (Addgene #12260) and pMD2.g (3,2 µg) (Addgene #12259) using X-tremeGene9 (48 µL) (Roche) according to the manufacturer's protocol. After 18 h incubation, media were replaced with 20 mL DMEM with 1.1 g/100 mL bovine serum albumin (no FBS) (Biowest). After another ~24 h of incubation, lentivector was harvested and pooled by collecting media in 50-mL conical tubes and centrifuged at 200 × *g* to remove remaining cell debris. Next, supernatant was aliquoted and stored at -80 °C.

Lentivector concentration was estimated by titrating six volumes between 0 and 750 µL onto 2 × 10⁶ cells in 2 mL medium (with FBS) followed by spinfection. Media also contained 8 µg/mL polybrene (Sigma-Aldrich) with the exception of SK-MM-1 cells since they didn't tolerate it. The next day, cells were harvested and divided over two 75-cm² flasks-one with and one without 1 µg/mL puromycin (Sigma-Aldrich). Cells were selected for 48-72 h until all non-transduced were dead. Cells in remaining flasks were counted and multicplicity of infection (MOI) was calculated for each virus volume by comparing counts in the puromycin-selected to the non-selected condition.

### Example 3. Cas9 stable cell line generation

DS and RL SpCas9 expressing cells were generated by stable spinfection of the Lenti-Cas9-2A-Blasti lentiviral construct (Addgene #73310) followed by blasticidin (Gibco, 10 µg/mL) selection. Population-level Cas9 expression was first assessed by western blot with a Cas9 antibody (NBP2-36440, Novus, 1:2500). Next, knockout efficiency was checked by lentiviral transduction of a sgRNA targeting the essential *XPO1* gene and monitoring cell death in Cas9 expressing cells compared to the parental cell line. Finally, we transduced a construct expressing in-frame EGFP together with an EGFP targeting sgRNA and monitored loss of EGFP fluorescence over time with the IncuCyte (Sartorius). For both cell lines, polyclonal cells were used for subsequent CRISPR screening.

### Example 4. CRISPR-Cas9 knockout screening

To establish IC values for screening, cells were seeded in 150-cm² flasks and treated with selinexor at different concentrations for four days after which live cells were counted and compared to an untreated control flask. HAP1, KMS-28BM and SK-MM-1 cells were screened with the one-vector library encoding both sgRNA and Cas9 while for DS and RL cells, the two-vector library, which uses two separate vectors for the sgRNA and Cas9, was used. Cells were transduced at a MOI of approximately 0.25 and a coverage of >200-fold. Puromycin-selected cells were allowed to recover for 4-7 days after which selection with selinexor started. HAP1, KMS-28BM, SK-MM-1, DS and RL cells were selected with selinexor at IC₅₀, corresponding to a concentration of 100 nM, 125 nM, 140 nM, 200 nM and 40 nM, respectively. In addition, KMS-28BM were also screened at IC₂₀, corresponding to a concentration of 40 nM. Finally, for every cell line, a no-drug condition was included (DMSO). For each condition, three parallel replicates containing at least 20 × 10⁶ cells each were initiated after puromycin selection and maintained independently throughout screening and downstream processing. Cells were passaged every 2-4 days and cell pellets of 20 × 10⁶ were collected at regular intervals. Screening continued for >20 cell doublings, more precisely until day 24, 42, 28, 28 and 25 for HAP1, KMS-28BM, SK-MM-1, DS and RL cells, respectively.

gDNA was extracted from cell pellets with the QIAamp Blood Maxi Kit (Qiagen) and further concentrated using ethanol and NaCl precipitation. sgRNA cassettes were amplified in two consecutive PCR reactions: The first reaction to amplify sgRNA regions and the second to further amplify the sgRNA spacer sequence and attach TruSeq adapters with i5 and i7 barcodes. DNA libraries were sequenced on an Illumina HiSeq4000 or HiSeq X.

Sequencing analysis was performed in R with the CRISPRBetaBinomial method⁵². This method utilizes a beta-binomial distribution to model sgRNA counts, a modified Student's t-test for to compare sgRNA levels and Fisher's combined probability test to estimate gene-level significance. Vulcano plots were created with Graphpad Prism 9. From the HAP1 screen, the top 13 genes with highest positive or negative log₂(fold change) (log2FC) and p-value <0.01 were selected for individual validation.

### Example 5. Generation of single knockout cell lines

For individual hit validation, we used the GPP sgRNA Design tool to select the two top-ranking knockout sgRNAs not present in the Brunello library⁶. We also selected three random safe-targeting guides which were previously shown to have no impact on cell viability beyond toxicity from on-target DNA damage⁵³. Individual sgRNAs were cloned into plentiCRISPRv2 (Addgene #52961) following the standard protocol⁵⁴. Forward and reverse guides were ordered as single-stranded DNA oligonucleotides (Integrated DNA Technologies) containing the 20 bp spacer sequence with overhangs at the 3' and 5' ends to enable ligation. After hybridization and phosphorylation, double-stranded oligonucleotides were ligated into the BsmBI-digested (Thermo Fisher Scientific) plentiCRISPRv2 backbone. The ligation mixture was transformed into Stbl3 chemically competent *E. coli* (Invitrogen) after which individual colonies were picked up and inoculated into 5 mL LB medium. Plasmid DNA was extracted with the Nucleospin Plasmid Transfection-grade Mini kit (Macherey-Nagel) and correct integration of the insert was checked with Sanger sequencing. Next, lentivector was produced in a single, separate 75-cm² flask for each sgRNA, as described above.

To generate single-knockout cells, HAP1 and KMS-28BM cells were transduced with a single sgRNA lentivector at an MOI of approximately 0.25. Transduced cells were selected with 1 µg/mL puromycin for 2-3 days until the no-lentivector control was completely dead.

### Cell confluency assay

Cells were seeded into 96-well plates at 2500 cells/well (HAP1) in 100 µL medium. The next day, 100 µL of medium with selinexor was added to obtain a final concentration of 100 nM. Cell confluency was measured with IncuCyte (Sartorius) at the indicated time. After each scan, cells were detached and equal volumes of each well were transferred to a new 96-well plate with fresh medium. This process was repeated up until the final scan. Relative cell confluency was calculated as the ratio of cell confluency in the selinexor-treated condition to the corresponding DMSO control.

Statistical analysis was performed with Graphpad Prism 9 software. Data points from safe-targeting sgRNAs were pooled, as well as those from single gene knockouts. Both conditions were compared at the indicated time point using a two-sided Student's t-test.

### Example 6. MTS cell viability assay

HAP1 cells were seeded in 96-well plates at 2500 cells/well in 100 µL medium. The next day, a serial dilution of compound dissolved in 100 µL medium was added, with nine different final concentrations as indicated. After three days of incubation, 20 µL of MTS reagent (CellTiter 96 Aqueous Non-Radioactive Cell Proliferation Assay from Promega) was added and cells were incubated for an additional 2-4 h after which absorbance was measured at 490 nm with a Safire 2 microplate reader (Tecan). For each experiment, absorbance measurements of three replicate wells were averaged. Wells containing only growth medium were used for blank correction. Relative cell viability for a given concentration was obtained as the ratio of blank-corrected absorbance in treated to untreated cells. Similarly, KMS-28BM cells were seeded at 7000 cells/well and viability was measured after six days of incubation. Dose-response curves and IC₅₀ values were obtained by plotting values with Graphpad Prism 9 and fitting a non-linear, four-parameter regression curve. IC₅₀ values were compared using an extra sum-of-squares F-test, which tests whether fitting multiple models with a different IC₅₀ values for different conditions is a significant improvement over using a global model with a single, shared IC₅₀ value for all conditions.

### Example 7. Gene set enrichment analysis of patient data

We reanalyzed previously described RNA sequencing data obtained from bone marrow samples from patients treated with selinexor and dexamethasone on the Phase 2b, open-label, single-arm, registrational STORM study²⁶. Fastq files were adapter trimmed using Trim Galore v0.6.6 and subsequently aligned to the human genome GRCh38 using STAR aligner v2.7.9a with two-pass mode⁵⁵. Gene counts were obtained using HTSeq-Count v0.13.5⁵⁶ and then normalized with DESeq2 v1.34.0⁵⁷. Pathway analysis was performed by calculating the single sample gene set enrichment analysis score for each sample in each pathway of the MSigDB Reactome⁵⁸⁻⁶⁰ pathway set using the GSVA R package v1.38.2 with the kernel estimation of the cumulative density function set to Poisson⁶¹, then running a Cox proportional hazards model for progression-free survival (PFS) using all patients for each pathway. PFS was also compared between patients separated into those with high and low activity of indicated pathways, using an optimal cut point determined by the maxstat R package v0.7-25 to stratify patients, and log-rank tests to calculate p-values^{62,63}.

### Example 8. Western blot

For the experiments monitoring XPO1 degradation, HAP1 cells were seeded in 6-well plates at 100,000 cells/mL. The next day, cells were treated with selinexor and/or bortezomib as indicated. The pan-caspase inhibitor Q-VD-OPh was added to all conditions at 10 µM final concentration to delay onset of apoptosis. Cells were treated with compound overnight or as indicated. Next, cells were harvested, washed with ice-cold PBS and lysed in RIPA buffer (Thermo Fisher Scientific) with 1× HALT Protease Inhibitor Cocktail (Thermo Fisher Scientific) on ice for 20 min. Cell debris was removed by centrifugation at 20.000 × *g* and 4 °C for 10 min and total protein content was quantified with the Pierce BCA Protein Assay kit (Thermo Fisher Scientific). We used an automated, capillary-based Simple Western size assay (Wes, ProteinSimple) to detect and quantify our proteins of interest. 5 µL of lysate at a final concentration of 200 µg/mL was loaded onto the assay plate. For detection, the following primary antibodies and dilutions were used: CRM1/XPO1 (NB100-79802, Novus, 1:1000), TUBB (NB600-936, Novus, 1:40) and FLAG (F4049, Sigma-Aldrich, 1:250). HRP-conjugated secondary antibodies were used for chemiluminescent detection. Run and data analysis was performed with Compass for Simple Western software (ProteinSimple).

### Example 9. Immunoprecipitations

The protocol for lysis and co-immunoprecipitation was modified from an earlier report⁶⁴. HEK293T cells were seeded in 75-cm² flasks at 3×10⁶ cells/flask. The next day, cells were (co-)transfected with 8 µg pDNA encoding FLAG-ASB8, FLAG-luc2, KPNA3-FLAG and/or XPO1 using 24 µL of TurboFectin 8.0 (OriGene). 24 h later, media were changed with fresh media containing 10 nM bortezomib, 10 µM Q-VD-OPh and selinexor at the indicated concentration. After 15 min of incubation, cells were harvested and counted. For each condition, 10×10⁶ cells were collected by centrifugation at 200 × *g* for 5 min. Cells were washed with 10 mL ice-cold PBS and centrifuged again. Next, cell pellets were lysed in 500 µL freshly prepared buffer (20 mM Tris-HCl pH=8, 150 mM NaCl, 10% glycerol, 1% Triton X-100, 1 mM EDTA, 1 mM PMSF, 1 mM NaF, 1 mM DTT, 1 mM Na₃VO₄ and 1 × HALT Protease Inhibitor Cocktail) and incubated on a rotary tube mixer at 4 °C for 20 min. Lysates were cleared by centrifugation at 20.000 × *g* and 4 °C for 10 min. 50 µL of the supernatant was stored separately at -20 °C (whole-cell lysate). The remaining supernatant was pre-cleared by incubation with 50 µL Dynabeads Protein A (Thermo Fisher Scientific) on a rotary tube mixer at 4 °C for 4 h. Afterwards, the non-bound fraction was incubated with 20 µL anti-FLAG M2 magnetic beads (Sigma-Aldrich) at 4 °C overnight. Supernatant was discarded and beads were washed twice: a first time with high-salt lysis buffer (300 mM NaCl) and a second time with normal lysis buffer. Bound proteins were eluted by boiling in 25 µL 1× Sample Buffer (ProteinSimple) with 1% SDS at 95 °C for 10 min. Whole-cell lysates and eluates were directly subjected to Simple Wes analysis.

### Example 10. Immunofluorescence staining

A stable FLAG-ASB8 expressing HAP1 cell line was established by lentiviral transduction of the coding sequence. In a similar fashion, control cells were transduced with the same lentiviral vector harboring a 300 bp non-coding RNA sequence (stuffer). Cells were seeded in chambered coverslips, allowed to adhere and subsequently treated with either 200 nM selinexor or DMSO overnight. Cells were fixed with 4% paraformaldehyde in PBS and permeabilized with 0.2% Triton-X in PBS. Staining was performed with a primary FLAG antibody (Sigma-Aldrich, F1804, 1:500) and a secondary Alexa Fluor 488 goat anti-mouse antibody (Invitrogen, A11029, 1:500) while cell nuclei were counterstained with DAPI. Cells were imaged by confocal laser scanning microscopy (Leica Microsystems)

### Example 11. HAP1 endogenous XPO1 C528S mutant cell line

A HAP1 cell line with an endogenous *XPO1* C528S mutation was established using our previously reported CRISPR-mediated homology-directed repair method^{21,65}. Cells were cotransfected with plasmids expressing Cas9-NLS, a sgRNA targeting the *XPO1* C528 locus) and a ssDNA repair template containing the C528S missense mutation. After electroporation with a Neon Electroporation device (Thermo Fisher Scientific) and a recovery period of three days, cells were selected with 1 µM selinexor. Surviving cells constitute a polyclonal mixture of hetero- and homozygous *XPO1* C528S mutants.

### References

1. Housman, G. et al. Drug Resistance in Cancer: An Overview. Cancers 6, 1769-1792 (2014).
2. Schwaederle, M. et al. Association of Biomarker-Based Treatment Strategies With Response Rates and Progression-Free Survival in Refractory Malignant Neoplasms. JAMA Oncol 2, 1452 (2016).
3. Mali, P. et al. RNA-guided human genome engineering via Cas9. Science 339, 823-6 (2013).
4. Cong, L. et al. Multiplex genome engineering using CRISPR/Cas systems. Science 339, 819-23 (2013).
5. Shalem, O. et al. Genome-scale CRISPR-Cas9 knockout screening in human cells. Science 343, 84-87 (2014).
6. Doench, J. G. et al. Optimized sgRNA design to maximize activity and minimize off-target effects of CRISPR-Cas9. Nat Biotechnol 34, 184-191 (2016).
7. Jinek, M. et al. A programmable dual-RNA-guided DNA endonuclease in adaptive bacterial immunity. Science 337, 816-821 (2012).
8. Estoppey, D. et al. Identification of a novel NAMPT inhibitor by CRISPR/Cas9 chemogenomic profiling in mammalian cells. Sci Rep 7, 42728 (2017).
9. Ding, Y. et al. Synthetic lethality between HER2 and transaldolase in intrinsically resistant HER2-positive breast cancers. Nat Commun 9, 4274 (2018).
10. Colic, M. et al. Identifying chemogenetic interactions from CRISPR screens with drugZ. Genome Med 11, 1-12 (2019).
11. Szlachta, K. et al. CRISPR knockout screening identifies combinatorial drug targets in pancreatic cancer and models cellular drug response. Nat Commun 9, 4275 (2018).
12. Milton, C. K. et al. A genome-scale CRISPR screen identifies the ERBB and mTOR signaling networks as key determinants of response to PI3K inhibition in pancreatic cancer. Mol Cancer Ther 19, 1423-1435 (2020).
13. Wang, C. et al. Genome-wide CRISPR screens reveal synthetic lethality of RNASEH2 deficiency and ATR inhibition. Oncogene 38, 2451-2463 (2019).
14. Zimmermann, M. et al. CRISPR screens identify genomic ribonucleotides as a source of PARP-trapping lesions. Nature 559, 285-289 (2018).
15. Lin, K. H. et al. P2RY2-AKT activation is a therapeutically actionable consequence of XPOL inhibition in acute myeloid leukemia. Nat Cancer 3, 837-851 (2022).
16. Fornerod, M., Ohno, M., Yoshida, M. & Mattaj, I. W. CRM1 Is an Export Receptor for Leucine-Rich Nuclear Export Signals. Cell 90, 1051-1060 (1997).
17. Azmi, A. S., Uddin, M. H. & Mohammad, R. M. The nuclear export protein XPOL - from biology to targeted therapy. Nat Rev Clin Oncol 18, 152-169 (2021).
18. Daelemans, D. et al. A synthetic HIV-1 Rev inhibitor interfering with the CRM1-mediated nuclear export. Proceedings of the National Academy of Sciences 99, 14440-14445 (2002).
19. Van Neck, T. et al. Inhibition of the CRM1-mediated nucleocytoplasmic transport by N-azolylacrylates: Structure-activity relationship and mechanism of action. Bioorg Med Chem 16, 9487-9497 (2008).
20. Kalid, O., Toledo Warshaviak, D., Shechter, S., Sherman, W. & Shacham, S. Consensus Induced Fit Docking (cIFD): methodology, validation, and application to the discovery of novel Crm1 inhibitors. J Comput Aided Mol Des 26, 1217-1228 (2012).
21. Neggers, J. E. et al. Identifying Drug-Target Selectivity of Small-Molecule CRM1/XPO1 Inhibitors by CRISPR/Cas9 Genome Editing. Chem Biol 22, 107-116 (2015).
22. Neggers, J. E. et al. Target identification of small molecules using large-scale CRISPR-Cas mutagenesis scanning of essential genes. Nat Commun 9, 502 (2018).
23. Tai, Y. T. et al. CRM1 inhibition induces tumor cell cytotoxicity and impairs osteoclastogenesis in multiple myeloma: Molecular mechanisms and therapeutic implications. Leukemia 28, 155-165 (2014).
24. Lapalombella, R. et al. Selective inhibitors of nuclear export show that CRM1/XPO1 is a target in chronic lymphocytic leukemia. Blood 120, 4621-4634 (2012).
25. Etchin, J. et al. KPT-330 inhibitor of CRM1 (XPO1)-mediated nuclear export has selective anti-leukaemic activity in preclinical models of T-cell acute lymphoblastic leukaemia and acute myeloid leukaemia. Br J Haematol 161, 117-127 (2013).
26. Chari, A. et al. Oral Selinexor-Dexamethasone for Triple-Class Refractory Multiple Myeloma. New England Journal of Medicine 381, 727-738 (2019).
27. Grosicki, S. et al. Once-per-week selinexor, bortezomib, and dexamethasone versus twice-per-week bortezomib and dexamethasone in patients with multiple myeloma (BOSTON): a randomised, open-label, phase 3 trial. The Lancet 396, 1563-1573 (2020).
28. Kalakonda, N. et al. Selinexor in patients with relapsed or refractory diffuse large B-cell lymphoma (SADAL): a single-arm, multinational, multicentre, open-label, phase 2 trial. Lancet Haematol 7, e511-e522 (2020).
29. Richard, S. et al. Selinexor, bortezomib, and dexamethasone versus bortezomib and dexamethasone in previously treated multiple myeloma: Outcomes by cytogenetic risk. Am J Hematol 96, 1120-1130 (2021).
30. Hing, Z. A. et al. Selinexor is effective in acquired resistance to ibrutinib and synergizes with ibrutinib in chronic lymphocytic leukemia. Blood 125, 3128-3132 (2015).
31. Ranganathan, P. et al. XPO1 inhibition using selinexor synergizes with chemotherapy in acute myeloid leukemia by targeting DNA repair and restoring topoisomerase IIα to the nucleus. Clinical Cancer Research 22, 6142-6152 (2016).
32. Cui, Y. et al. The Synergistic Effect of Melphalan and XPO1 Inhibition in Pre-Clinical Models of Multiple Myeloma. Blood 128, 5662 (2016).
33. Muqbil, I. et al. Anti-tumor activity of selective inhibitor of nuclear export (SINE) compounds, is enhanced in non-Hodgkin lymphoma through combination with mTOR inhibitor and dexamethasone. Cancer Lett 383, 309-317 (2016).
34. Argueta, C. et al. Synergistic Anti-Tumor Effect of KPT-8602, a Second Generation Selective Inhibitor of Nuclear Export (SINE) Compound, and Panobinostat, a Pan-Histone Deacetylase (HDAC) Inhibitor in Multiple Myeloma. Blood 128, 3298 (2016).
35. Shang, E. et al. Dual Inhibition of Bcl-2/Bcl-xL and XPO1 is synthetically lethal in glioblastoma model systems. Sci Rep 8, 15383 (2018).
36. Nishida, Y. et al. Dual Inhibition of MDM2 and XPO1 Induces Synergistic Apoptosis in Wild-type p53 Acute Myeloid Leukemia Through Nuclear Accumulation of p53 and Suppression of c-Myc. Clin Lymphoma Myeloma Leuk 19, S215 (2019).
37. Fischer, M. A. et al. Venetoclax response is enhanced by selective inhibitor of nuclear export compounds in hematologic malignancies. Blood Adv 4, 586-598 (2020).
38. Owusu-Ansah, F., Afaghani, J., Lee, S. & Taylor, J. Inhibiting the Nuclear Exporter XPO1 and the Antiapoptotic Factor BCL2 Is Synergistic in XPO1 Mutant and Wildtype Lymphoma. Blood 136, 17-18 (2020).
39. Wang, J. et al. XPO1 inhibition synergizes with PARP1 inhibition in small cell lung cancer by targeting nuclear transport of FOXO3a. Cancer Lett 503, 197-212 (2021).
40. Verbeke, D. et al. The XPO1 inhibitor KPT-8602 synergizes with dexamethasone in acute lymphoblastic Leukemia. Clinical Cancer Research 26, 5747-5758 (2020).
41. Marijon, H. et al. Selinexor, a selective inhibitor of nuclear export, enhances the anti-tumor activity of olaparib in triple negative breast cancer regardless of BRCA1 mutation status. Oncotarget 12, 1749 (2021).
42. Inoue, A. et al. Sequential Administration of XPO1 and ATR Inhibitors Enhances Therapeutic Response in TP53-mutated Colorectal Cancer. Gastroenterology 161, 196-210 (2021).
43. Govaerts, I. et al. PSEN1-selective gamma-secretase inhibition in combination with kinase or XPO-1 inhibitors effectively targets T cell acute lymphoblastic leukemia. Journal of Hematology & Oncology 2021 14:1 14, 1-14 (2021).
44. Brinton, L. T. et al. Cotargeting of XPO1 Enhances the Antileukemic Activity of Midostaurin and Gilteritinib in Acute Myeloid Leukemia. Cancers (Basel) 12, 1574 (2020).
45. Taylor, J. et al. Altered nuclear export signal recognition as a driver of oncogenesis. Cancer Discov 9, 1452-1467 (2019).
46. Lagana, A. et al. E2F1 Is a Biomarker of Selinexor Resistance in Relapsed/Refractory Multiple Myeloma Patients. Blood 132, 3216-3216 (2018).
47. Chari, A. et al. Results of the Pivotal STORM Study (Part 2) in Penta-Refractory Multiple Myeloma (MM): Deep and Durable Responses with Oral Selinexor Plus Low Dose Dexamethasone in Patients with Penta-Refractory MM. Blood 132, 598-598 (2018).
48. Miyake, T. M. et al. NRG1/ERBB3 pathway activation induces acquired resistance to XPO1 inhibitors. Mol Cancer Ther 19, 1727-1735 (2020).
49. Restrepo, P. et al. Transcriptomic Correlates of Response to Selinexor in Multiple Myeloma Reveal a Predictive Signature. Blood 138, 457-457 (2021).
50. Andresen, C. A. et al. Protein interaction screening for the ankyrin repeats and suppressor of cytokine signaling (SOCS) box (ASB) family identify asb11 as a novel endoplasmic reticulum resident ubiquitin ligase. Journal of Biological Chemistry 289, 2043-2054 (2014).
51. Hart, T. et al. Evaluation and Design of Genome-Wide CRISPR/SpCas9 Knockout Screens. G3: Genes|Genomes|Genetics 7, 2719 (2017).
52. Jeong, H.-H., Kim, S. Y., Rousseaux, M. W. C., Zoghbi, H. Y. & Liu, Z. Beta-binomial modeling of CRISPR pooled screen data identifies target genes with greater sensitivity and fewer false negatives. Genome Res 29, 999-1008 (2019).
53. Morgens, D. W. et al. Genome-scale measurement of off-target activity using Cas9 toxicity in high-throughput screens. Nat Commun 8, (2017).
54. Sanjana, N. E., Shalem, O. & Zhang, F. Improved vectors and genome-wide libraries for CRISPR screening. Nat Methods 11, 783-784 (2014).
55. Dobin, A. et al. STAR: ultrafast universal RNA-seq aligner. Bioinformatics 29, 15-21 (2013).
56. Anders, S., Pyl, P. T. & Huber, W. HTSeq-a Python framework to work with high-throughput sequencing data. Bioinformatics 31, 166-169 (2015).
57. Love, M. I., Huber, W. & Anders, S. Moderated estimation of fold change and dispersion for RNA-seq data with DESeq2. Genome Biol 15, 1-21 (2014).
58. Fabregat, A. et al. The Reactome Pathway Knowledgebase. Nucleic Acids Res 46, D649-D655 (2018).
59. Liberzon, A. et al. Molecular signatures database (MSigDB) 3.0. Bioinformatics 27, 1739-1740 (2011).
60. Subramanian, A. et al. Gene set enrichment analysis: A knowledge-based approach for interpreting genome-wide expression profiles. Proceedings of the National Academy of Sciences 102, 15545-15550 (2005).
61. Hänzelmann, S., Castelo, R. & Guinney, J. GSVA: Gene set variation analysis for microarray and RNA-Seq data. BMC Bioinformatics 14, 1-15 (2013).
62. Hothorn, T. & Lausen, B. On the exact distribution of maximally selected rank statistics. Comput Stat Data Anal 43, 121-137 (2003).
63. Lausen, B., Hothorn, T., Bretz, F. & Schumacher, M. Assessment of Optimal Selected Prognostic Factors. Biometrical Journal 46, 364-374 (2004).
64. Martin, A. P. et al. STK 38 kinase acts as XPO 1 gatekeeper regulating the nuclear export of autophagy proteins and other cargoes. EMBO Rep 20, (2019).
65. Neggers, J. E. et al. Heterozygous mutation of cysteine528 in XPOL is sufficient for resistance to selective inhibitors of nuclear export. Oncotarget 7, 68842-68850 (2016).
66. Walker, C. J. et al. Preclinical and clinical efficacy of XPO1/CRM1 inhibition by the karyopherin inhibitor KPT-330 in Ph+ leukemias. Blood 122, 3034-3044 (2013).
67. Than, H. et al. Coordinated inhibition of nuclear export and Bcr-Abl1 selectively targets chronic myeloid leukemia stem cells. Leukemia 2020 34:6 34, 1679-1683 (2020).
68. Matthews, L. et al. Reactome knowledgebase of human biological pathways and processes. Nucleic Acids Res 37, (2009).
69. Bateman, A. et al. UniProt: the universal protein knowledgebase in 2021. Nucleic Acids Res 49, D480-D489 (2021).
70. Collart, M. A. & Panasenko, O. O. The Ccr4-Not Complex: Architecture and Structural Insights. Subcell Biochem 83, 349-379 (2017).
71. White, E. J. F., Matsangos, A. E. & Wilson, G. M. AUF1 regulation of coding and noncoding RNA. Wiley Interdiscip Rev RNA 8, e1393 (2017).
72. Huang, J., Li, S. J., Chen, X. H., Han, Y. & Xu, P. hnRNP-R regulates the PMA-induced c-fos expression in retinal cells. Cell Mol Biol Lett 13, 303-311 (2008).
73. Cappelli, S., Romano, M. & Buratti, E. Systematic analysis of gene expression profiles controlled by hnRNP Q and hnRNP R, two closely related human RNA binding proteins implicated in mRNA processing mechanisms. Front Mol Biosci 5, 79 (2018).
74. Jiao, W., Datta, J., Lin, H. M., Dundr, M. & Rane, S. G. Nucleocytoplasmic Shuttling of the Retinoblastoma Tumor Suppressor Protein via Cdk Phosphorylation-dependent Nuclear Export. Journal of Biological Chemistry 281, 38098-38108 (2006).
75. Holien, T. & Sundan, A. The role of bone morphogenetic proteins in myeloma cell survival. Cytokine Growth Factor Rev 25, 343-350 (2014).
76. Niimi, H., Pardali, K., Vanlandewijck, M., Heldin, C. H. & Moustakas, A. Notch signaling is necessary for epithelial growth arrest by TGF-β. Journal of Cell Biology 176, 695-707 (2007).
77. Blokzijl, A. et al. Cross-talk between the Notch and TGF-β signaling pathways mediated by interaction of the Notch intracellular domain with Smad3. Journal of Cell Biology 163, 723-728 (2003).
78. Ikushima, H. & Miyazono, K. TGFbeta signalling: a complex web in cancer progression. Nat Rev Cancer 10, 415-424 (2010).
79. Oulès, B. et al. Contribution of GATA6 to homeostasis of the human upper pilosebaceous unit and acne pathogenesis. Nat Commun 11, 5067 (2020).
80. Huang, T. T., Kudo, N., Yoshida, M. & Miyamoto, S. A nuclear export signal in the N-terminal regulatory domain of IκBα controls cytoplasmic localization of inactive NF-κB/IκBα complexes. Proceedings of the National Academy of Sciences 97, 1014-1019 (2000).
81. Rodriguez, M. S., Thompson, J., Hay, R. T. & Dargemont, C. Nuclear Retention of IκBα Protects It from Signal-induced Degradation and Inhibits Nuclear Factor κB Transcriptional Activation. Journal of Biological Chemistry 274, 9108-9115 (1999).
82. Johnson, C., Van Antwerp, D. & Hope, T. J. An N-terminal nuclear export signal is required for the nucleocytoplasmic shuttling of Ikappa Balpha. EMBO J 18, 6682-6693 (1999).
83. Kashyap, T. et al. Efficacy of Selinexor Is Dependent on IκB-α Expression and NF-Kb Deactivation in Multiple Myeloma Cells. Blood 128, 5660-5660 (2016).
84. Turner, J. G. et al. XPO1 inhibitor combination therapy with bortezomib or carfilzomib induces nuclear localization of IκB-α and overcomes acquired proteasome inhibitor resistance in human multiple myeloma. Oncotarget 7, 78896-78909 (2016).
85. Kashyap, T. et al. Selinexor, a Selective Inhibitor of Nuclear Export (SINE) compound, acts through NF-εcB deactivation and combines with proteasome inhibitors to synergistically induce tumor cell death. Oncotarget 7, 78883-78895 (2016).
86. Kotarba, G., Krzywinska, E., Grabowska, A. I., Taracha, A. & Wilanowski, T. TFCP2/TFCP2L1/UBP1 transcription factors in cancer. Cancer Lett 420, 72-79 (2018).
87. Dienstmann, R., Rodon, J., Serra, V. & Tabernero, J. Picking the Point of Inhibition: A Comparative Review of PI3K/AKT/mTOR Pathway Inhibitors. Mol Cancer Ther 13, 1021-1031 (2014).
88. Hermida, M. A., Dinesh Kumar, J. & Leslie, N. R. GSK3 and its interactions with the PI3K/AKT/mTOR signalling network. Adv Biol Regul 65, 5-15 (2017).
89. Gardam, S., Sierro, F., Basten, A., Mackay, F. & Brink, R. TRAF2 and TRAF3 Signal Adapters Act Cooperatively to Control the Maturation and Survival Signals Delivered to B Cells by the BAFF Receptor. Immunity 28, 391-401 (2008).
90. Leotoing, L. et al. A20-binding Inhibitor of Nuclear Factor-κB (NF-κB)-2 (ABIN-2) Is an Activator of Inhibitor of NF-εcB (IκB) Kinase α (IKKo)-mediated NF-κB Transcriptional Activity. Journal of Biological Chemistry 286, 32277-32288 (2011).
91. DepMap: The Cancer Dependency Map Project at Broad Institute. https://depmap.org/portal/.
92. Li, R. et al. E3 ligase ASB8 promotes porcine reproductive and respiratory syndrome virus proliferation by stabilizing the viral Nsp1α protein and degrading host IKKβ kinase. Virology 532, 55-68 (2019).
93. Guo, Y. et al. E3 ubiquitin ligase ASB8 negatively regulates interferon via regulating TBK1/IKKi homeostasis. Mol Immunol 121, 195-203 (2020).
94. Rath, O. & Kozielski, F. Kinesins and cancer. Nat Rev Cancer 12, 527-539 (2012).
95. Prieto, G., Fullaondo, A. & Rodriguez, J. A. Prediction of nuclear export signals using weighted regular expressions (Wregex). Bioinformatics 30, 1220-1227 (2014).
96. Xu, D. et al. LocNES: a computational tool for locating classical NESs in CRM1 cargo proteins. Bioinformatics 31, 1357 (2015).
97. Vercruysse, T. et al. The Second-Generation Exportin-1 Inhibitor KPT-8602 Demonstrates Potent Activity against Acute Lymphoblastic Leukemia. Clinical Cancer Research 23, 2528-2541 (2017).
98. Watanabe, M., Masuyama, N., Fukuda, M. & Nishida, E. Regulation of intracellular dynamics of Smad4 by its leucine-rich nuclear export signal. EMBO Rep 1, 176-182 (2000).
99. Kιrlι, K. et al. A deep proteomics perspective on CRM1-mediated nuclear export and nucleocytoplasmic partitioning. Elife 4, (2015).
100. Piñol-Roma, S. HnRNP proteins and the nuclear export of mRNA. Semin Cell Dev Biol 8, 57-63 (1997).
101. Blaxall, B. C. et al. Differential expression and localization of the mRNA binding proteins, AU-rich element mRNA binding protein (AUF1) and Hu antigen R (HuR), in neoplastic lung tissue. Mol Carcinog 28, 76-83 (2000).
102. Carpenter, B. et al. The roles of heterogeneous nuclear ribonucleoproteins in tumour development and progression. Biochimica et Biophysica Acta (BBA) - Reviews on Cancer 1765, 85-100 (2006).
103. Perrotti, D. & Harb, J. G. BCR-ABL1 kinase-dependent alteration of mRNA metabolism: Potential alternatives for therapeutic intervention. Leuk Lymphoma 52, 30-44 (2011).
104. De Keersmaecker, K. et al. Exome sequencing identifies mutation in CNOT3 and ribosomal genes RPL5 and RPL10 in T-cell acute lymphoblastic leukemia. Nat Genet 45, 186-190 (2013).
105. Vicente, C. et al. The CCR4-NOT complex is a tumor suppressor in Drosophila melanogaster eye cancer models. J Hematol Oncol 11, 108 (2018).
106. Xu, D., Grishin, N. V. & Chook, Y. M. NESdb: a database of NES-containing CRM1 cargoes. Mol Biol Cell 23, 3673-3676 (2012).
107. Hart, T. et al. High-Resolution CRISPR Screens Reveal Fitness Genes and Genotype-Specific Cancer Liabilities. Cell 163, 1515-1526 (2015).
108. Wang, T., Wei, J. J., Sabatini, D. M. & Lander, E. S. Genetic screens in human cells using the CRISPR-Cas9 system. Science 343, 80-4 (2014).
109. Drabavicius, G. & Daelemans, D. Intermedilysin cytolytic activity depends on heparan sulfates and membrane composition. PLoS Genet 17, e1009387 (2021).
110. Parrish, P. C. R. et al. Discovery of synthetic lethal and tumor suppressor paralog pairs in the human genome. Cell Rep 36, 109597 (2021).
111. Thompson, N. A. et al. Combinatorial CRISPR screen identifies fitness effects of gene paralogues. Nat Commun 12, 1302 (2021).
112. Crochiere, M. L. et al. A method for quantification of exportin-1 (XPO1) occupancy by Selective Inhibitor of Nuclear Export (SINE) compounds. Oncotarget 7, 1863-1877 (2016).
113. Fung, H. Nuclear export receptor CRM1 recognizes nuclear export signals with diverse confomations (Doctoral dissertation). (2017).
114. Wang, P. et al. Repression of classical nuclear export by S-nitrosylation of CRM1. J Cell Sci 122, 3772-3779 (2009).

## Claims

1. A method of identifying a compound that promotes ASB (Ankyrin repeat and SOCS box protein) mediated degradation of a target protein, comprising the step of:
- providing in vitro conditions allowing the binding of a compound and the protein target, and
- determining whether binding of the compound to the target protein results in the binding of an ASB protein to the target protein,
wherein a compound that results in the binding of an ASB protein to a target protein with the compound, is a candidate medicinal compound for conditions wherein a target protein is to be degraded.

2. The method according to claim 1, wherein the ASB is ASB8.

3. The method according to claim 1 or 2, further comprising the step of testing whether the binding of the compound to the target protein inhibits the function of the target protein.

4. The method according to any one of claims 1 to 3, which determining the binding of an ASB protein to a target protein is performed with a co-immune precipitation assay with an antibody against the target protein, and wherein is determined whether the ASB protein is co-precipitated.

5. The method according to any one of claims 1 to 3, wherein binding between an ASB protein and a target protein is determined by a method selected from the group consisting of native mass spectrometry, FRET, HTFR and a thermal shift assay.

6. The method according to any one of claims 1 to 3, wherein a target protein is selected by a method comprising the steps of:
- providing a cell line wherein the ASB gene is inactivated and a cell line wherein the ASB protein is overexpressed,
- treating the cell lines with a promiscuous metabolite,
- determining the identity and concentration of proteins in the treated cell lines,
wherein a protein, which is absent in the ABS overexpressing cells or has a lower concentration in the ABS overexpressing cells, is candidate target protein.

7. The method according to claim 6, wherein the promiscuous metabolite is 4-octyl-itacanoate.

8. The method according to any one of claims 1 to 3, comprising the step of
- providing a cell line wherein ASB gene is inactivated and a cell line wherein ASB protein is overexpressed,
- adding a compound to the cell lines, and
- determining a change in the phenotype of one of the cell lines, wherein a change of phenotype is indicative of the compound being a compound promoting ASB mediation degradation.

9. The method according to claim 8, further determining the target protein that binds the compound added to the cell lines.

10. The method according to claim 9, further determining whether the compound modified or inhibits the function of the target protein.
